# EUROPEAN PATENT APPLICATION

(11) **EP 2 233 502 A1**
(43) Date of publication of application: **29.09.2010**
(21) Application number: 09156520.0
(22) Date of filing: 27.03.2009
(51) Int. Cl.: C07K 16/34, C07K 16/18, C07K 14/415, A61K 39/395

(54) **Sialylated antigen-specific antibodies for treatment or prophylaxis of unwanted inflammatory immune reactions and methods of producing them**

(71) Applicant: Deutsches Rheuma-Forschungszentrum Berlin, 10117 Berlin (DE)
(72) Inventor: Ehlers, Marc, 10117 Berlin (DE); Hess, Constanze, 10405 Berlin (DE); Lorenz, Alexandra, 10367 Berlin (DE); Winkler, André, 14469 Potsdam (DE)
(74) Representative: Gulde Hengelhaupt Ziebig & Schneider

(57) **Abstract**

The present invention is directed to a method of producing a sialylated antigen-specific antibody specific for a human autoimmune antigen or allergen or human Rhesus factor D antigen, comprising an Fc-portion of IgG type, and exhibiting a sialic acid residue at the Fc-portion, to an antibody produced by said method and to a pharmaceutical composition comprising such an antibody.

## Description

### Field of the invention:

The present invention relates to novel antibodies and immune complexes for treatment or prophylaxis of unwanted inflammatory immune reactions, e.g. in autoimmune diseases, allergies and/or anti-Rhesus factor D reactions as well as to methods of producing such antibodies.

### Background of the invention:

Although progress has been made over the years, the treatment of autoimmune diseases such as rheumatoid arthritis, systemic lupus erythematosus, multiple sclerosis and diabetes mellitus type 1 still remain a field of high medical need.

It is well established that high doses of monomeric immunoglubulin G (IgG) purified from pooled human plasma, so called intravenous immunoglobulin or IVIG, confer anti-inflammatory activity in a variety of autoimmune settings. The immuno-modulatory effect of IVIG administration is unspecific in that it does not affect specifically a particular immune response but affects the immune system on a global level. This results in a decrease in the overall immune responsiveness of the patient. IVIG reveals an overall immunosuppressive effect, the treated individual is immune suppressed. In the case of patients with autoimmune disease, IVIG is administered at a high dose (generally 1-2 grams IVIG per kg body weight) to attempt to decrease the severity of the autoimmune disease. At present, the preferred dosage regimen for autoimmune diseases is 2 grams IVIG per kilogram of body weight for three to six months over a five day course once a month. Then maintenance therapy of 100 to 400 mg/kg of body weight every 3 to 4 weeks follows.

The anti-inflammatory activity of IVIG has been demonstrated in a variety of animal models, including antibody ITP (A. Samuelson, T.L. Towers, J.V. Ravetch, Science 291, 484 (2001)) and nephrotoxic nephritis (Y. Kaneko, F. Nimmerjahn, J.V. Ravetch, J. Exp. Med. 203, 789 (2006)). It could be demonstrated that its anti-inflammatory effect is a property of the Fc-portion of the purified IgG in IVIG preparations and of its associated glycans. Removal of the terminal sialic acid residues from IVIG or its papain-derived Fc-portion abrogates the anti-inflammatory activity (Y. Kaneko, F. Nimmerjahn, J.V. Ravetch, Science 313, 670 (2006)).

Recently it could be shown that a sialylated recombinant human IgG Fc-portion is sufficient for the anti-inflammatory effect of IVIG or preparations enriched for sialylated IVIG (R.M. Anthony et al., Science 320, 373 (2008); WO 07/117505). Thus, the anti-inflammatory effect of IVIG is independent from the antigen specificity of the IgG molecules present in the respective IVIG preparation. In order to achieve the global anti-inflammatory effect of IVIG it is not necessary that the immunoglobulins comprise variable domains at all.

By identifying the anti-inflammatory effect to be a function solely of the sialylated Fc-portion of an IgG, the therapeutic dose for treatment of autoimmune disease could be lowered from 1-2 g/kg for IVIG, over 0.1 g/kg for enriched IVIG for sialylated IgG, to 30 mg/kg for recombinant sialylated IgG Fc-portion (R.M. Anthony et al., Science 320, 373 (2008)). Thus, although significant reduction of required dosage has already been achieved, there remains the need to find means for therapeutic treatment of autoimmune diseases that are effective at even lower doses. Furthermore, there remains a need for means to treat autoimmune disease more specifically with less side effects especially on other parts of the immune system.

The problem to be solved by the present invention can be seen in the provision of means that diminish, avoid or overcome one or more of the drawbacks of the state of the art.

### Description of the invention:

The present invention solves the problem by providing novel sialylated antigen-specific antibodies that can be used for treatment or prophylaxis of unwanted inflammatory immune reactions, in particular for autoimmune diseases or allergies or anti-Rhesus factor D reactions.

In one aspect the invention provides a method of producing a sialylated antibody, specific for a human autoimmune antigen, allergen or human Rhesus factor D antigen, comprising an Fc-portion of IgG type, and exhibiting a sialic acid residue at the Fc-portion, comprising the following steps:
a) isolation of B cells from an individual suffering from an autoimmune disease, allergy or exhibiting an immune response to human Rhesus factor D antigen and individualisation of isolated B cells;
b) optionally, cloning of selected B cells;
c) optionally, selection of B cell clone that provides native antibody specific for human Rhesus factor D antigen or an antigen associated with autoimmune disease or allergy of donor of B cells;
d) isolation of the whole or part of the nucleic acid coding sequence encoding the native antibody provided by an individualized B cell or a cell clone originating from an individualized B cell and being specific for human Rhesus factor D antigen or an antigen associated with autoimmune disease or allergy of donor of B cells;
e) use of isolated native antibody nucleic acid coding sequence as a whole or in part to provide a nucleic acid coding sequence encoding an antibody with specificity for the same antigen as the respective native antibody and exhibiting an Fc-portion of IgG type, preferably of human IgG type;
f) expression of antibody encoded by the nucleic acid coding sequence of step e); and
g) addition of sialic acid residue to the Fc-portion of the expressed antibody.

This method leads to production of antibodies which have beneficial properties. Such antibodies may be used in treatment or prophylaxis of e.g. autoimmune disease or allergy or anti-Rhesus factor D reactivity. Because of its antigen specificity, the immune-modulatory effect of the antibody of the invention is specific in that it does primarily affect a particular immune response, but does not affect the immune system on a global level. The antibody of the invention is effective for treatment of autoimmune disease or allergy or anti-Rhesus factor D reactivity at very low concentrations.

### In the following some definitions of terms of the invention are provided:

### Antibody:

For the purpose of the present invention, the term antibody is not limited to an antibody with the classical heavy and light chain architecture. The terms "antibody" or "antibodies" as used herein are art-recognized terms and are understood to refer to molecules or active fragments of molecules that bind to given antigens, particularly to immunoglobulin molecules and to immunologically active portions of immunoglobulin molecules, i.e molecules that contain a binding site that specifically binds an antigen. An immunoglobulin is a protein comprising one or more polypeptides substantially encoded by the immunoglobulin kappa and lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Also subclasses of the heavy chain are known. For example, IgG heavy chains in humans can be any of IgG1, IgG2, IgG3 and IgG4 subclass. The antibody of the invention can be of IgG class or any subclass thereof (IgG1, IgG2, IgG3, IgG4).

A typical antibody structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively.

Antibodies exist as full length intact antibodies or as a number of well-characterized fragments produced by digestion with various peptidases or chemicals. Thus, for example, pepsin digests an antibody below the disulfide linkages in the hinge region to produce F(ab')2, a dimer of Fab which itself is a light chain joined to VH-CH1 by a disulfide bond. The F(ab')2 may be reduced under mild conditions to break the disulfide linkage in the hinge region thereby converting the F(ab')2 dimer into two Fab' monomers. The Fab' monomer is essentially a Fab fragment with part of the hinge region (see, Fundamental Immunology, W. E. Paul, ed., Raven Press, N.Y. (1993), for a more detailed description of other antibody fragments). While various antibody fragments are defined in terms of the digestion of an intact antibody, one of skill will appreciate that any of a variety of antibody fragments may be synthesized de novo either chemically or by utilizing recombinant DNA methodology. Thus, the term antibody, as used herein also includes antibody fragments either produced by the modification of whole antibodies or synthesized de novo or antibodies and fragments obtained by using recombinant DNA methodologies. "Antibodies" are intended within the scope of the present invention to include monoclonal antibodies, polyclonal antibodies, chimeric, single chain, bispecific, simianized, human and humanized antibodies as well as active fragments thereof. Examples of active fragments of molecules that bind to known antigens include separated light and heavy chains, Fab, Fab/c, Fv, Fab', and F(ab')2 fragments, including the products of an Fab immunoglobulin expression library and epitope-binding fragments of any of the antibodies and fragments mentioned above.

These active fragments can be derived from an antibody of the present invention by a number of techniques. For example, monoclonal antibodies can be cleaved with an enzyme, such as pepsin, and subjected to HPLC gel filtration. The appropriate fraction containing Fab fragments can then be collected and concentrated by membrane filtration and the like. For further description of general techniques for the isolation of active fragments of antibodies, see for example, Khaw, B. A. et al. J. Nucl. Med. 23:1011-1019 (1982); Rousseaux et al. Methods Enzymology, 121 :663-69, Academic Press, 1986.

Recombinantly made antibodies may be conventional full length antibodies, active antibody fragments known from proteolytic digestion, unique active antibody fragments such as Fv or single chain Fv (scFv), domain deleted antibodies, and the like. An Fv antibody is about 50 Kd in size and comprises the variable regions of the light and heavy chain. A single chain Fv ("scFv") polypeptide is a covalently linked VH:: VL heterodimer which may be expressed from a nucleic acid including VH- and VL-encoding sequences either joined directly or joined by a peptide-encoding linker. See Huston, et al. (1988) Proc. Nat. Acad. Sci. USA, 85:5879-5883. A number of structures are known for converting the naturally aggregated, but chemically separated light and heavy polypeptide chains from an antibody V region into an scFv molecule which will fold into a three dimensional structure substantially similar to the structure of an antigen-binding site. See, e.g. U.S. Patent Nos. 5,091,513, 5,132,405 and 4,956,778.

The combining site refers to the part of an antibody molecule that participates in antigen binding. The antigen binding site is formed by amino acid residues of the N-terminal variable ("V") regions VDJ and VJ of the heavy ("H") and light ("L") chains. The antibody variable regions comprise three highly divergent stretches referred to as "hypervariable regions" or "complementarity determining regions" (CDRs) which are interposed between more conserved flanking stretches known as "framework regions" (FRs). In an antibody molecule, the three hypervariable regions of a light chain (LCDR1, LCDR2, and LCDR3) and the three hypervariable regions of a heavy chain (HCDR1, HCDR2 and HCDR3) are disposed relative to each other in three dimensional space to form an antigen binding surface or pocket. The antibody combining site therefore represents the amino acids that make up the CDRs of an antibody and any framework residues that make up the binding site pocket.

The identity of the amino acid residues in a particular antibody that make up the combining site can be determined using methods well known in the art. For example, antibody CDRs may be identified as the hypervariable regions originally defined by Kabat et al. (see, "Sequences of Proteins of Immunological Interest," E. Kabat et al., ., U.S. Department of Health and Human Services; Johnson, G and Wu, TT (2001) Kabat Database and its applications: future directions. Nucleic Acids Research, 29: 205-206; http://immuno.bme.nwa.edu). The positions of the CDRs may also be identified as the structural loop structures originally described by Chothia and others, (see Chothia and Lesk, J. Mol. Biol. 196, 901 (1987), Chothia et al., Nature 342, 877 (1989), and Tramontano et al., J. Mol. Biol. 215, 175 (1990)). Other methods include the "AbM definition" which is a compromise between Kabat and Chothia and is derived using Oxford Molecular's AbM antibody modeling software (now Accelrys) or the "contact definition" of CDRs by Macallum et al., ("Antibody-antigen interactions: contact analysis and binding site topography," J Mol Biol. 1996 Oct I I;262(5):732-45).

The identity of the amino acid residues in a particular antibody that are outside the CDRs, but nonetheless make up part of the combining site by having a side chain that is part of the lining of the combining site (i.e., it is available to linkage through the combining site), can be determined using methods well known in the art such as molecular modeling and X-ray crystallography. See e.g., Riechmann et al., (1988) Nature, 332:;323-327.

Chimeric antibodies are those in which one or more regions of the antibody are from one species of animal and one or more regions of the antibody are from a different species of animal. A preferred chimeric antibody is one which includes regions from a primate immunoglobulin. A chimeric antibody for human clinical use is typically understood to have variable regions from a non-human animal, e.g. a rodent, with the constant heavy and light chain regions from a human. In contrast, a humanized antibody uses CDRs from the non-human antibody with most or all of the variable framework regions and all the constant regions from a human immunoglobulin. A chimeric antibody may include some changes to a native amino acid sequence of the human constant regions and the native non-human variable region sequence. Chimeric and humanized antibodies may be prepared by methods well known in the art including CDR grafting approaches (see, e.g., U.S. Patent Nos. 5,843,708; 6,180,370; 5,693,762; 5,585,089; 5,530,101), chain shuffling strategies (see e.g., U.S. Patent No. 5,565,332; Rader et al., Proc. Natl. Acad. Sci. USA (1998) 95:8910-8915), molecular modelling strategies (U.S. Patent No. 5,639,641), and the like.

A "humanized antibody" as used herein in the case of a two chain antibody is one where at least one chain is humanized. A humanized antibody chain has a variable region where one or more of the framework regions are human. A humanized antibody which is a single chain is one where the chain has a variable region where one or more of the framework regions are human. The non-human portions of the variable region of the humanized antibody chain or fragment thereof is derived from a non-human source, particularly a non-human antibody. The non-human contribution to the humanized antibody is typically provided in form of at least one CDR region which is interspersed among framework regions derived from one (or more) human immunoglobulin (s). In addition, framework support residues may be altered to preserve binding affinity.

Humanized antibody of reduced immunogenicity refers to a humanized antibody exhibiting reduced immunogenicity relative to the parent antibody, e.g., the murine antibody.

Humanized antibody substantially retaining the binding properties of the parent antibody refers to a humanized antibody which retains the ability to specifically bind the antigen recognized by the parent antibody used to produce such humanized antibody. Preferably the humanized antibody will exhibit the same or substantially the same antigen-binding affinity and avidity as the parent antibody. Ideally, the affinity of the antibody will not be less than 10% of the parent antibody affinity, more preferably not less than about 30%, and most preferably the affinity will not be less than 50% of the parent antibody. Methods for assaying antigen-binding affinity are well known in the art and include half-maximal binding assays, competition assays, and Scatchard analysis. Suitable antigen binding assays are described in this application.

The humanized antibody may further comprise constant regions (e.g., at least one constant region or portion thereof, in the case of a light chain, and preferably three constant regions in the case of a heavy chain). The constant regions of a humanized antibody if present generally are human.

Methods to obtain "humanized antibodies" are well known to those skilled in the art. (see, e.g., Queen et al., Proc. Natl Acad Sci USA, 86:10029-10032 (1989), Hodgson et al., Bio/Technology, 9:421 (1991)).

A "humanized antibody" may also be obtained by a novel genetic engineering approach that enables production of affinity-matured human-like polyclonal antibodies in large animals such as, for example, rabbits and mice. See, e.g. U.S. Pat No. 6,632,976.

The term constant region (CR) as used herein refers to constant regions genes of the immunoglobulin. The constant region genes encode the portion of the antibody molecule which confers effector functions. For Chimeric human antibodies and humanized antibodies, typically non-human (e.g., murine), constant regions are substituted by human constant regions. The constant regions of the subject chimeric or humanized antibodies are typically derived from human immunoglobulins. The heavy chain constant region can be selected from any of the five isotypes: alpha, delta, epsilon, gamma or mu. Further, heavy chains of various subclasses (such as the IgG subclasses of heavy chains) are responsible for different effector functions and thus, by choosing the desired heavy chain constant region, antibodies with desired effector function can be produced. Constant regions that may be used within the scope of this invention are gamma 1 (IgG1), gamma 2 (IgG2), gamma 3 (IgG3) and gamma 4 (IgG4) particularly an corresponding Fc region of the gamma 1 (IgG1), gamma 2 (IgG2), gamma 3 (IgG3) and/or gamma 4 (IgG4) isotype. The light chain constant region can be of the kappa or lambda type, preferably of the kappa type. In one embodiment the light chain constant region is the human kappa constant chain (Heiter et al. (1980) Cell 22:197-207) and the heavy constant chain is the human IgG4 constant chain.

The term "monoclonal antibody" is also well recognized in the art and refers to an antibody that is mass produced in the laboratory and originally comes from one B cell. Monoclonal antibodies are typically made by fusing a normally short-lived, antibody-producing B cell with a fast-growing cell, such as a cancer cell (sometimes referred to as an "immortal" cell) to generate a rapidly dividing hybridoma clone or by transient or stable transfection of a host organism, e.g. a cell line, yeast or bacterial strain, with an expression system encompassing a coding sequence for the desired antibody. The resulting host, hybrid cell, or hybridoma produces large quantities of the antibody. For the purpose of the present invention, "monoclonal antibody" is also to be understood to comprise antibodies that are produced by a mother clone which has not yet reached full monoclonality.

The antibody comprising an Fc-portion of IgG type includes those in which specific amino acid substitutions, additions or deletions are introduced into a native or parental sequence through the use of recombinant DNA techniques to modify the genes encoding the heavy chain constant region. The introduction of these modifications follows well- established techniques of molecular biology, as described in manuals such as Molecular Cloning (Sambrook and Russel, (2001)).

As used herein "specifically binds" or "specific for" in reference to an antibody means that the antibody binds to its target antigen with greater affinity than it does to a structurally different antigen(s).

### Antigen:

The term "antigen" refers to an entity or fragment thereof which can bind to an antibody. An antigen refers to an entity or fragment which can elicit an immune response in an organism, particularly an animal, more particularly a mammal including a human. The term antigen includes regions known as antigenic determinants or epitopes which refers to a portion of the antigen (which are contacted or which play a significant role in supporting a contact reside in the antigen responsible for antigenicity or antigenic determinants.

### Autoimmune antigen:

The term "autoimmune antigen" refers to antigens that are associated with an autoimmune disease. An autoimmune antigen associated with a specific autoimmune disease is an entity or fragment, e.g. a protein or complex of proteins, DNA or RNA, that is recognized by the immune system of at least a fraction of individuals suffering from said specific autoimmune disease. These antigens should, under normal healthy conditions, not be the target of the immune system. But, due to mainly genetic and/or environmental factors, the normal immunological tolerance for such an antigen has been lost in individuals suffering from a specific autoimmune disease associated with said antigen.

### Allergen:

The term "allergen" refers to a non-parasitic, in most cases environmental antigen capable of stimulating a hypersensitivity reaction in individuals. Most humans mount significant Immunoglobulin E (IgE) responses only as a defense against parasitic infections. However, some individuals mount an IgE response against common environmental antigens, the allergens.

### Rhesus factor D (or RhD) antigen:

Individuals either have, or do not have, the Rhesus factor D (or Rhesus factor, RhD) antigen on the surface of their red blood cells. This is usually indicated by 'RhD positive' (does have the RhD antigen) or 'RhD negative' (does not have the antigen) suffix to the ABO blood type. Unlike the ABO antigens, the only ways IgG antibodies are developed against the RhD are by transfusion or feto-maternal transfusion during pregnancy. That is, if a person who is RhD-negative has never been exposed to the RhD antigen, they do not possess the RhD antibody. There may be prenatal danger to the RhD-positive foetus when a pregnant woman is RhD-negative but the biological father is RhD-positive with the induction of unwanted anti-RhD specific IgG antibodies by the mother leading to hemolytic disease of the newborn and also of further RhD-positive foetus. Maternal IgG anti-RhD antibodies can pass through the placenta. The vast majority of RhD disease is preventable in modern antenatal care by injections of anti-RhD IgG antibodies. Protective anti-RhD IgG antibodies are generated by injection of RhD-positive erythrocytes in RhD-negative healthy human. Serum of these IgG anti-RhD antibody producing male volunteers are pooled, total IgG containing protective anti-RhD IgG is purified and used for prophylaxis of corrresponding pregnant woman.

In Table 1 below preferred examples of antigens are given. Each antigen is presented together with the associated disease and wherever appropriate the accession No. of the antigen is also provided.

### Fc portion of IgG type:

The term "Fc portion of IgG type" is used to define a C-terminal region of an immunoglobulin heavy chain of IgG type, irrespective of the subclass. The "Fc portion of IgG type" may be a native sequence Fc region or a variant Fc region. Although the boundaries of the Fc region of an immunoglobulin heavy chain might vary, the human IgG heavy chain Fc region is usually defined to stretch from an amino acid residue at position Cys226 or Pro230 in the hinge region, to the carboxyl-terminus thereof containing the CH2 and CH3 domain of the heavy chain.

The term "hinge region" is generally defined as stretching from GIu216 to Pro230 of human IgG1 (D.R. Burton, Mol Immunol 22, 161 (1985)). Hinge regions of other IgG isotypes may be aligned with the IgG1 sequence by placing the first and last cysteine residues forming inter-heavy chain S--S bonds in the same positions.

The "CH2 domain" of a human IgG Fc portion (also referred to as "Cγ2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain (D.R. Burton, Mol Immunol 22, 161 (1985)).

The "CH3 domain" comprises the stretch of residues C- terminal to a CH2 domain in an Fc portion (i.e., from about amino acid residue 341 to about amino acid residue 447 of an IgG).

A "native sequence Fc portion" comprises an amino acid sequence identical to the amino acid sequence of an Fc portion found in nature. A "variant Fc portion" as appreciated by one of ordinary skill in the art comprises an amino acid sequence which differs from that of a native sequence Fc portion by virtue of at least one amino acid modification. Preferably, the variant Fc portion has at least one amino acid substitution compared to a native sequence Fc portion or to the Fc portion of a parent polypeptide, e.g., from about one to about ten amino acid substitutions, and preferably from about one to about five amino acid substitutions in a native sequence Fc portion or in the Fc portion of the parent antibody. The variant Fc portion herein will preferably possess at least about 80% homology with a native sequence Fc portion and/or with an Fc portion of a parent antibody, and more preferably at least about 90% homology therewith, more preferably at least about 95% homology therewith, even more preferably, at least about 99% homology therewith.

Throughout the present specification and claims, the numbering of the residues in an immunoglobulin heavy chain is that of the EU index as in E.A. Kabat et al., Sequences of Proteins of Immunological Interest, 5th Ed. Public Health Service, National Institutes of Health, Bethesda, Md. (1991), which is expressly incorporated herein by reference. The "EU index as in Kabat" refers to the residue numbering of the human IgG1 EU antibody.

Sequences of human IgG1, IgG2, IgG3 and IgG4 are available under the following accession Nos (of IMGT (www.imgt.org)):
J00228 for human IgG1, also referred to as SEQ ID No. 14;
J00230 for human IgG2, also referred to as SEQ ID No. 15;
X03604 for human IgG3, also referred to as SEQ ID No. 16;and
K01316 for human IgG4, also referred to as SEQ ID No. 17;.

The amino acid sequence of a suitable human IgG1 constant region is given below, comprising CH1, hinge, CH2 and CH3 domain (Accession Number J00228, of IMGT (www.imgt.org)):
**CH1 (AS 118-215), referred to as SEQ ID No. 1:**
   ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKV
**Hinge (AS 216-230), referred to as SEQ ID No. 2:**
   EPKSCDKTHTCPPCP
**CH2 (AS 231-340), referred to as SEQ ID No. 3:**
   APELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAK
**CH3 (AS 341-447), referred to as SEQ ID No. 4:**
   GQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK

The term "native" or "parent" refers to an unmodified antibody comprising an Fc amino acid sequence. The parent antibody may comprise a native sequence Fc portion or an Fc portion with pre-existing amino acid sequence modifications (such as additions, deletions and/or substitutions).

The method of producing a sialylated antibody, specific for a human autoimmune antigen, allergen or human Rhesus factor D antigen, comprising an Fc-portion of IgG type, and exhibiting a sialic acid residue at the Fc-portion, comprises the following steps:

### a) isolation of B cells from an individual suffering from an autoimmune disease, allergy or exhibiting an immune response to human Rhesus factor D antigen and individualisation of isolated B cells.

B cells are preferably isolated from an individual suffering from an autoimmune disease. Non-limiting examples of autoimmune diseases are acute disseminated encephalomyelitis (ADEM), Addison's disease, Alopecia areata, Antiphospholipid antibody syndrome (APS), Autoimmune hemolytic anemia, Autoimmune hepatitis, Bullous pemphigoid, Coeliac disease, Crohns Disease, Dermatomyositis, Diabetes mellitus type 1, Goodpasture's syndrome, Graves' disease, Guillain-Barré syndrome (GBS), Hashimoto's disease, Idiopathic thrombocytopenic purpura, systemic Lupus erythematosus (SLE), Mixed Connective Tissue Disease, Multiple sclerosis (MS), Myasthenia gravis, Pemphigus Vulgaris, Pernicious anaemia, Polymyositis, Primary biliary cirrhosis, Rheumatoid arthritis (RA), Scleroderma, Sjögren's syndrome, Temporal arteritis (also known as "giant cell arteritis"), Ulcerative Colitis, Vasculitis, and Wegener's granulomatosis.

B cells can be isolated from one ore more individuals. Individuals are single organisms, preferably mammal, most preferably human or rodent organisms, like e.g. mouse or rat. An individual suffering from an autoimmune disease or allergy can be a human patient with a diagnosed autoimmune disease or allergy. An individual suffering from an autoimmune disease can also be a mammal suitable to be used in an animal model for an autoimmune disease or an allergy in another species, like e.g. in a human. Preferably the mammal is a rodent, e.g. a mouse or rat.

An individual with anti-human Rhesus factor reactive B cells can be a Rhesus factor negative woman who has born a Rhesus factor positive baby, a Rhesus factor negative male volunteer who has got Rhesus factor positive erythrocytes or an animal who has been immunized with appropriate human Rhesus factor positive erythrocytes or human Rhesus factor peptides to induce anti-human Rhesus factor antibodies.

Preferably memory B cells are isolated in a manner so that the isolated cells are viable, can be taken into culture and propagated for a certain amount of time. B cells are usually isolated under sterile conditions. If B cells are isolated form humans, the cells are isolated in accordance with good medical practice. The skilled person is aware of techniques that allow appropriate isolation of B cells from an individual. E.g. fluorescence activated cell sorting (FACS) or magnetic cell separation (MACS) are suitable methods for isolation of viable B cells from cell mixtures.

Isolated B cells are individualised so that it is possible to access single individual B cells. Preferably isolated B cells are individualised e.g. by dilution or directly during FACS sorting and transfer into an appropriate vessel, e.g. a culture vessel like a multi well plate, so that a single B cell is placed into one well of the multi well plate. In an exemplified method, B cells are singularised, seeded with a density of 1 cell per well in culture vessels and may be cultured under appropriate conditions.

Alternatively, unsorted or antigen-specific single memory B cells or plasma blasts or plasma cells are isolated and afterwards directly used to prepare cDNA comparable to the method described recently (Tiller, J Immunol Methods, 329, 112-124 (2008)). In this case the skilled person continues directly with step d).

### b) Optionally, cloning of isolated B cells.

The method of the invention is not limited to a special way of cloning B cells. In principle any method for cloning B cells is applicable, as long as B cells are kept under conditions under which the cells grow for a certain period of time and provide antibodies. Methods for cloning B cells are well known in the art. An isolated and individualized memory B cell might be stimulated with eg. cytokines, Toll-like receptor ligands and/or antigen, or is fused with a fast-growing cell, such as cancer cell (sometimes referred to as an "immortal" cell) to generate a rapidly dividing hybridoma clone, or is transfected with EBV to generate immortalized cells.

### c) Optionally, selection of B cell clone that provides native antibody specific for human Rhesus factor D antigen or an antigen associated with autoimmune disease or allergy of donor of B cells.

B cell clones are identified and selected that provide an antibody specific for a predetermined antigen which is associated with an autoimmune disease or allergy of the donor of the B cells. Basically antibody fractions of B cell clones are checked for specific antigen-antibody interaction with a predetermined antigen, preferably an antigen associated with an autoimmune disease or allergy or Rhesus factor D reactivity, more preferably the antigen is selected from table 1. The method of the invention is not limited to a particular method for identification and/or selection of B cell clones. Many methods for identification and/or selection of B cell clones providing antibodies with a desired specificity are known to the person skilled in the art. The skilled person has no difficulties in selecting an appropriate method. Testing for specific interaction of antibody fractions of B cell clones with predetermined antigens can be done e.g. by ELISA, Immunoblotting or other methods.

### d) Isolation of the whole or part of the nucleic acid coding sequence encoding the native antibody provided by an individualized B cell or a cell clone originating from an individualized B cell and being specific for human Rhesus factor D antigen or an antigen associated with autoimmune disease or allergy of donor of B cells.

Once a B cell clone has been identified and selected, the whole or part of the coding sequence encoding the native antibody provided by that B cell clone is isolated by recombinant DNA methods (e.g. Molecular Cloning, Sambrook and Russel, 2001). Since the basic structure of the gene(s) encoding an antibody molecule is known in most of the organisms, the skilled person has no difficulties in isolating at least the variable VDJ heavy chain part and the variable VJ light chain part by PCR and sequencing them (Tiller, J Immunol Methods, 329, 112-124 (2008)). Preferably, at least the nucleic acid sequence encoding the CDR region of the native antibody is isolated and determined.
Alternatively, the whole or part of the coding sequence encoding the native antibody provided by an isolated single memory B cell, plasma blast or plasma cell is derived by single cell RT-PCR. The respective coding sequence can also be derived from genomic DNA of a single B cell e.g. by PCR and subsequent cloning and/or sequencing.

### e) Use of isolated native antibody nucleic acid coding sequence as a whole or in part to provide a nucleic acid coding sequence encoding an antibody with specificity for the same antigen as the respective native antibody and exhibiting an Fc-portion of IgG type, preferably of human IgG type.

If appropriate, the isolated nucleic acid heavy and light chain coding sequences of a native antibody is used as a whole or in part to provide nucleic acid coding heavy and light chain sequences encoding an antibody with substantially the same binding specificity as the native antibody of the individualised isolated B cell. In case the constant parts of the heavy or light chain sequences are incomplete or improper, they can be substituted by separately cloned human IgG1, 2, 3 or 4 or human kappa or lambda sequences, respectively.
In case the native antibody is not of IgG type, or does not exhibit a desired Fc-portion of IgG type, the native antibody nucleic acid coding sequence needs to be genetically engineered in order to provide a coding sequence encoding an antibody with substantially the same binding specificity as the isolated antibody and exhibiting an Fc-portion of IgG type, preferably of human IgG type.
Engineering of native antibody coding sequence may comprise only the replacement or addition of an Fc portion by a desired Fc portion.
Engineering may extend to settings where only the CDR or part of the hyper variable region of the CDR of the native antibody is used to prepare a coding sequence encoding an antibody of the invention. The CDR may be grafted on a different frame work. The antibody may be humanized. The CDR may be used to switch antibody format. It is recognized by one skilled in the art that such engineering may have an effect on the binding affinity of the resulting antibody. Engineered antibodies exhibit an affinity of not less than 10%, preferably not less than 30%, more preferably of not less than 50% of the parent antibody affinity.
Preferably the nucleic acid coding sequence of step e) encoding an antibody of the invention comprises a nucleic acid sequence encoding for an Fc-portion of IgG type comprising the amino acid sequences of SEQ ID Nos 3 and 4 of human IgG1 or corresponding sequences of human IgG2, 3 or 4.

### f) Expression of antibody using coding sequence of step e).

In order to express the antibody of the invention, the coding sequence of step e) will be cloned into an appropriate expression system. Many suitable vectors and expression systems are known to the person skilled in the art. E.g. the isolated heavy and light chain coding sequences of step e) may be cloned separately in two expression plasmids or both together in two expression cassettes of one expression plasmid suitable to express the heavy and light chain parts of an antibody.
The coding sequence of step e) encoding an antibody specific for an autoimmune antigen or allergen or Rhesus factor D antigen and comprising an Fc portion of IgG type will be expressed in order to produce said antibody protein. Expression can basically be performed in any host organism. Preferably antibody expression is performed in eucaryotic cells, more preferably in mammalian cells, in particular in mammalian cells of rodent or human origin. The antibody of the present invention can be expressed in a host expression system, i.e., host cells, capable of N-linked glycosylation. Typically, such host expression systems may comprise fungal, plant, vertebrate or invertebrate expression systems. In one embodiment the host cell is a mammalian cell, such as a Chinese hamster ovary (CHO) cell line, (e.g. CHO-KI; ATCC CCL-61), Green Monkey cell line (COS) (e.g. COS 1 (ATCC CRL-1650), COS 7 (ATCCCRL-1651)); mouse cell (e.g. NS/0), Baby Hamster Kidney (BHK) cell line (e.g. ATCC CRL-1632 or ATCC CCL-10), or human cell (e.g. HEK 293 (ATCC CRL-1573) or modifications thereof), or any other suitable cell line, e.g. available from public depositories such as the American Type Culture Collection, Rockville, Md.

Further, an insect cell line, such as a Lepidoptora cell line, e.g. Sf9, a plant cell line, a fungal cell line, e.g., yeast such as, for example, Saccharomyces cerevisiae, Pichia pastoris, Hansenula spp. It will be appreciated by one of ordinary skill in the art that in some cases modifications to host cells may be required to insure that N-linked glycosylation and glycan maturation occur to result in a complex, biantennary sugar as typically found on the Fc domain of human IgG (see for example S.R. Hamilton et al . Science, 313, 1441 (2006); H. Li, et al., Nature Biotechnology 24, 210 (2006); S. Wildt and T.U. Grengross Nature Reviews Microbiology 3, 119 (2005)).

### g) Addition of sialic acid residue to the Fc-portion of the expressed antibody.

This step is to ensure that the product of the method of the invention exhibits a sialic acid residue at the Fc portion of IgG origin and can be part of the antibody expression of step f). This can be e.g. the case, if the sialic acid is added during expression the antibody of the invention by the host. Alternatively or in addition, sialic acid may be attached to the expressed antibody in vitro.
Preferably the sialic acid residue or the sialic acid residues are attached to the antibody at a predetermined position of the Fc portion, in particular at position Asn²⁹⁷ or a homologue position of the Fc portion. For the purpose of this invention, a homologue position to Asn²⁹⁷ of the Fc portion refers to an Asn residue or another residue suitable for N-linked glycan modification at a position of the respective Fc portion that shows greatest homology to an amino acid sequence comprising 5 amino acids upstream and downstream of Asn²⁹⁷. Preferably the homologue position of the Fc-portion is a sequence motif of the Fc-portion comprising the amino acid sequence:
REEQYNSTYRV (also referred to as SEQ ID No. 5), and/or
REEQFNSTYRV (also referred to as SEQ ID No. 18),
wherein the residue suitable for N-linked glycan modification is underlined.

Preferably sialic acid residues are present on glycan structures of the Fc portion of IgG type, more preferably the glycan structure is N-linked to an amino acid of the Fc portion. The sialic acid residue can be linked to a galactose residue of the glycan structure, preferably in a 2,6 linkage as described in R.M. Anthony et al., Science 320, 373 (2008). In a preferred embodiment the sialic acid residue or two sialic acid residues are attached to the antibody of the invention at position Asn²⁹⁷ of the Fc portion via the following glycan structure: 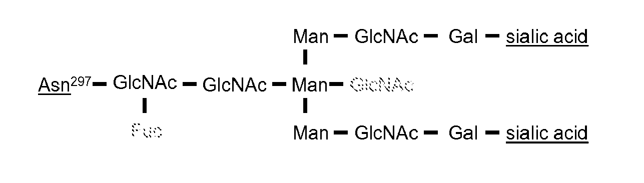 , wherein sugars Fuc and GIcNAc given in brackets are optional (Fuc: fucose; Man: Manose; GIcNAc: Acethylglucosamine; Gal: galactose).

The sialic acid residue(s) can be added during expression by the host cell. Therefore a host cell may be selected that expresses an β1,4-galactosyltransferase and/or α2,6-sialyltransferase. A desired host cell may be transfected in order to transiently or stably express one of these enzymes or both. Preferably a α2,6-sialyltransferase and/or a β1,4-galactosyltransferase of rodent, e.g. mouse or rat, or human origin is used for addition of sialic acid residues to the expressed antibody.

Alternatively sialic acid residues can be added to the expressed antibody in vitro, e.g. by incubation of the antibody with a sialyltransferase, preferably by a α2,6-sialyltransferase of rodent or human origin. More preferably, sialic acid residues can be added to the expressed antibody in vitro, e.g. by incubation of the antibody with a galactosyltransferase, preferably by a β1,4-galactosyltransferase and a sialyltransferase, preferably by a α2,6-sialyltransferase, both enzymes of preferentially human origin.

Preferentially, as many as possible antigen-specific IgG molecules are sialylated, which will reduce the amount of antigen-specific IgG to give to the patient.

The invention is also directed to another Method of producing a sialylated antibody specific for a human autoimmune antigen, allergen or human Rhesus factor D antigen, comprising an Fc-portion of IgG type, and exhibiting a sialic acid residue at the Fc-portion, comprising the following steps:
i) providing blood sample from an individual suffering from an autoimmune disease, allergy or exhibiting an immune response to human Rhesus factor D antigen or an autoimmune antigen or allergen;
ii) isolation of IgG antibody specific for human Rhesus factor D antigen or an autoimmune antigen or allergen from blood sample; and
iii) addition of sialic acid residue to the Fc-portion of the isolated IgG antibody.

Preferably the sialic acid is added to the antibody molecules in a way analogous to the method described in detail above.

Preferably an IgG antibody is isolated that is specific for an antigen selected from table 1.

The invention is also directed to an antibody obtainable by a method of the invention as described above.

The invention is also directed to an isolated antibody obtained or obtainable by a method of the invention as described above.

The invention is also directed to a polyclonal mixture of isolated IgG antibodies obtained or obtainable by a method of the invention as described above.

In another aspect, the present invention is directed to an antibody specific for an antigen selected from autoimmune antigens, allergens or rhesus D antigen, comprising an Fc-portion of IgG type, and exhibiting a sialic acid residue at the Fc-portion.

The antibody of the invention preferably exhibits a binding affinity of ≤ 10⁻⁶M for the antigen or allergen.

In a preferred embodiment, the Fc-portion of the antibody of the invention is of murine or human origin or has been humanised.

Preferably the antibody of the present invention exhibits an Fc-portion of IgG type comprising the amino acid sequences of SEQ ID Nos 3 and 4 of human IgG1 or corresponding sequences of human IgG2, 3 or 4.

In the antibody of the invention, the sialic acid residue can be positioned at amino acid Asn²⁹⁷ or homologue position of the Fc-portion. In a particular preferred embodiment, the antibody of the invention comprises a sialic acid residue at the Asparagine residue of the amino acid sequence motif of the Fc-portion:
REEQYNSTYRV (also referred to as SEQ ID No. 5), and/or
REEQFNSTYRV (also referred to as SEQ ID No. 18),
wherein the residue suitable for N-linked glycan modification is underlined.

Preferably, the antibody of the invention is specific for an antigen selected from table 1.

More preferably, the antibody of the invention is specific for an autoimmune antigen, e.g. an autoimmune antigen of table 1.

The present invention is also directed to an immune complex comprising an antibody of the invention specifically bound to its antigen.

An immune complex comprises or consists of a combination of an antigen with an antibody directed against that antigen. Thus the immune complex is the result of a specific antigen-antibody binding reaction. The bound antigen and the binding antibody are referred to as a single entity in this state. An immune complex may consist of one or two antigen molecules and one antibody molecule. The immune complex of the invention may comprise more than one antigen molecule and more than one antibody molecule of the same or different classes. The immune complex of the invention comprises preferably only one class of antigen molecules. The immune complex of the invention may comprise different classes of antibodies, wherein at least two antibody classes recognize different epitopes on the one antigen class. If the immune complex of the invention comprises more than one antibody class, at least one, more than one or all antibody classes of the immune complex are antibodies of the invention.

The invention is also directed to a pharmaceutical composition comprising an antibody of the invention, or an immune complex of the invention and a pharmaceutically acceptable excipient like e.g. a suitable carrier or diluent.

Preferably the antibody of the invention or the immune complex of the invention is an active ingredient of the pharmaceutical composition and/or is present in an effective amount.

The term "effective amount" denotes an amount of the antibody or immune complex of the invention, respectively, having a prophylactically or therapeutically relevant effect on a disease or pathological conditions. A prophylactic effect prevents the outbreak of a disease. A therapeutically relevant effect relieves to some extent one or more symptoms of a disease or returns to normal either partially or completely one or more physiological or biochemical parameters associated with or causative of the disease or pathological conditions. The respective amount for administering the antibody or immune complex, respectively, is sufficiently high in order to achieve the desired prophylactic or therapeutic effect. It will be understood by the skilled person that the specific dose level, frequency and period of administration to any particular mammal will depend upon a variety of factors including the activity of the specific components employed, the age, body weight, general health, sex, diet, time of administration, route of administration, drug combination, and the severity of the specific therapy. Using well-known means and methods, the exact amount can be determined by one of skill in the art as a matter of routine experimentation.

In the pharmaceutical composition of the invention at least 20% of the total antibody content is made of an antibody of the invention, preferably at least 50%, more preferably at least 75%, most preferable at least 95%.

When used for therapy or prophylaxis, the pharmaceutical composition will generally be administered as a formulation in association with one or more pharmaceutically acceptable excipients. The term "excipient" is used herein to describe any ingredient other than the antibody of the invention or the immune complex of the invention. The choice of excipient will to a large extent depend on the particular mode of administration. Excipients can be suitable carriers and/or diluents.

The pharmaceutical composition of the invention may be administered orally. Oral administration may involve swallowing, so that the composition enters the gastrointestinal tract, or buccal or sublingual administration may be employed by which the composition enters the blood stream directly from the mouth.

Formulations suitable for oral administration include: solid formulations such as tablets; capsules containing particulates, liquids, or powders; lozenges (including liquid-filled); and chews; multi- and nano-particulates; gels; solid solutions; liposomes; films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The pharmaceutical composition of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the antibody of the invention may make up from 1 weight % to 80 weight % of the dosage form, more typically from 5 weight % to 60 weight % of the dosage form. In addition to the antibody of the invention, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80 weight % antibody of the invention, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human use are typically pliable water-soluble or water-swellable thin film dosage forms which may be rapidly dissolving or mucoadhesive and typically comprise antibody of the invention, a film-forming polymer, a binder, a solvent, a humectant, a plasticiser, a stabiliser or emulsifier, a viscosity-modifying agent and a solvent. Some components of the formulation may perform more than one function.

The pharmaceutical composition may be water-soluble or insoluble. A water-soluble composition typically comprises from 1 weight % to 80 weight %, more typically from 20 weight % to 50 weight %, of the solutes. Less soluble compositions may comprise a greater proportion of the composition, typically up to 88 weight % of the solutes. Alternatively, the pharmaceutical composition may be in the form of multiparticulate beads.

The film-forming polymer may be selected from natural polysaccharides, proteins, or synthetic hydrocolloids and is typically present in the range from 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in U.S. Pat. No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of pharmaceutical composition of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLApoly(dl-lactic-coglycolic)acid (PGLA) microspheres.

The pharmaceutical composition of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated-see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (e.g. Powderject(TM), Bioject(TM), etc.) injection.

Formulations for topical administration may be formulated to be immediate and/or modified release. Modified release formulations include delayed-, sustained-, pulsed-, controlled-, targeted and programmed release.

The pharmaceutical composition of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 µg to 20 mg of the antibody or immune complex of the invention per actuation and the actuation volume may vary from 1 µl to 100 µl. A typical formulation may comprise an antibody of the invention, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA, modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 0.001 mg to 10 mg of the pharmaceutical composition of the invention. The overall daily dose will typically be in the range 0.001 mg to 40 mg of antibody or immune complex of the invention which may be administered in a single dose or, more usually, as divided doses throughout the day.

The pharmaceutical composition of the invention may be particularly suitable for an administration by inhalation.

The pharmaceutical composition of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The pharmaceutical composition of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The antibody, the immune complex and/or the pharmaceutical composition of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

For administration to human patients, the total daily dose of the antibody, the immune complex and/or the pharmaceutical composition of the invention is typically in the range 0.001 mg to 5000 mg depending, of course, on the mode of administration. For example, an intravenous daily dose may only require from 0.001 mg to 40 mg. The total daily dose may be administered in single or divided doses and may, at the physician's discretion, fall outside of the typical range given herein.

These dosages are based on an average human subject having a weight of about 65 kg to 70 kg. The physician will readily be able to determine doses for subjects whose weight falls outside this range, such as infants and the elderly.

The present invention is also directed to a medical formulation, e.g. one of the formulations mentioned above intended for medical use or application, comprising an antibody, an immune complex and/or a pharmaceutical composition of the invention.

The present invention also encompasses a kit comprising an antibody, an immune complex, a pharmaceutical composition or a medical formulation of the invention, optionally with written instructions for use and/or with means for administration.

In a further aspect the present invention is directed to an antibody, an immune complex, a pharmaceutical composition, a medical formulation or a kit of the invention for treatment and/or prophylaxis of autoimmune disease or allergy or anti-Rhesus factor D reaction. The terms "treatment" and "therapeutically," as used herein, refer to the act of treating, as "treating" is defined below. As used herein, the term "treating" refers to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein "treating" may also refer to preventing the recurrence of a disease, disorder or condition or of one or more symptoms associated with such disease, disorder or condition.

For treatment and/or prophylaxis of autoimmune disease or allergy or anti-Rhesus factor D reaction, irrespective of the route of administration, the antibody of the invention is administered at a daily dose per treatment cycle of not more than 20 mg/kg body weight, preferably of not more than 10 mg/kg body weight, more preferably selected from the range of 1µg/kg to 20 mg/kg body weight, most preferably selected from a range of 0.01 to 10mg/kg body weight. **FIGURES:**

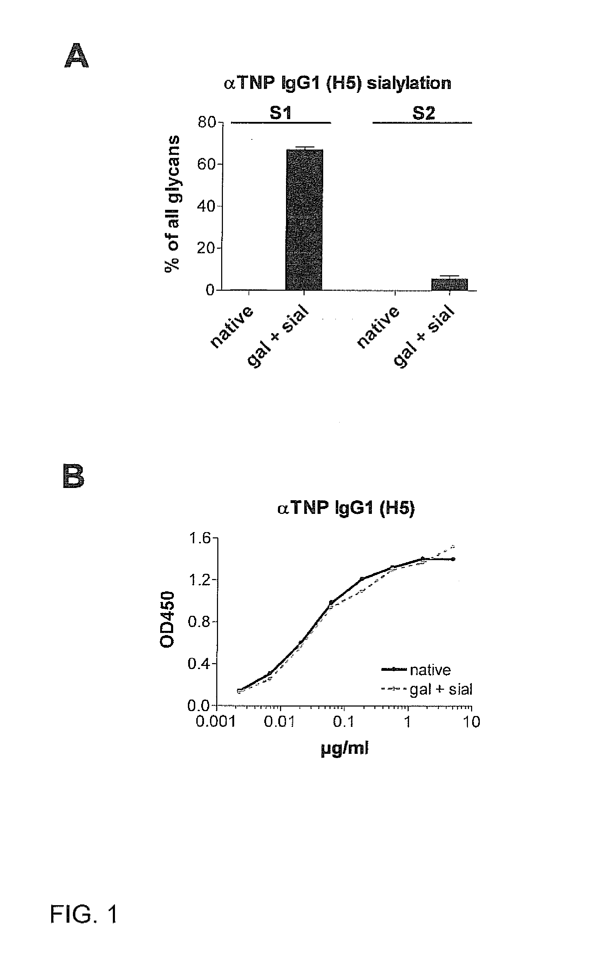

- FIG. 1: **(A)** shows results of in vitro galactosylation (gal) and sialylation (sial) of αTNP IgG (hybridoma clone H5); S1, one sialic acid; S2, two sialic acid; **(B)** shows that sialylation of H5 IgG did not alter binding properties of H5 IgG.

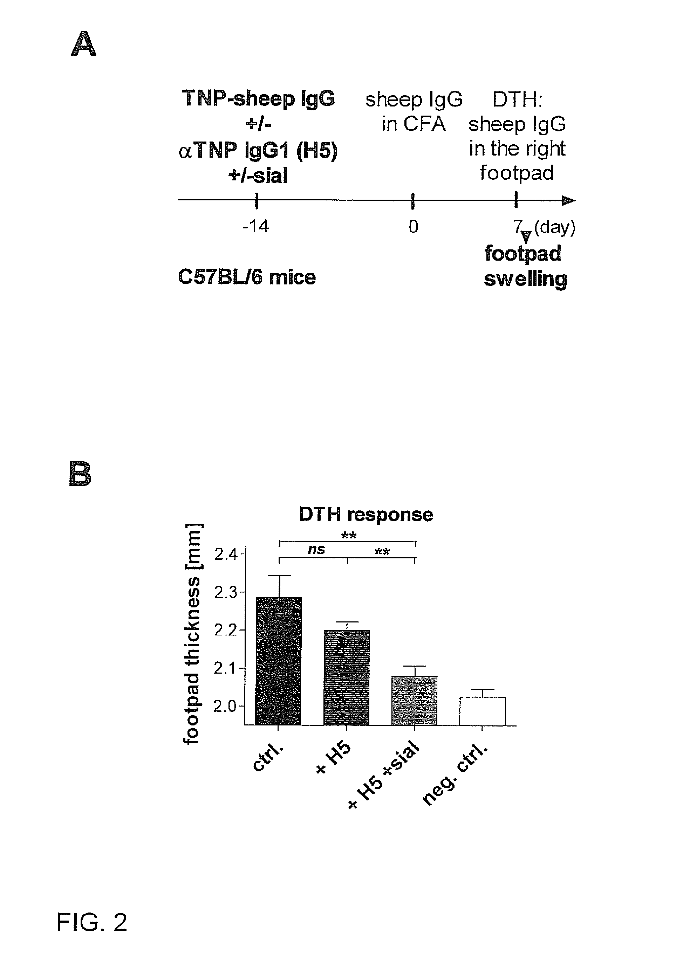

- FIG. 2: Immune complexes containing antigen plus sialylated IgGs protect from immune pathology (delayed type hypersensitivity (DTH) mouse model). **(A)** shows a schematic representation of the treatment course of the DTH model; **(B)** summarizes the results of the DTH model, wherein footpad thickness is given for the different treatments at day 5 after local DTH induction, ctrl. = TNP-sheep IgG (antigen) without H5 αTNP IgG antibody (n=5); + H5 = antigen + non-sialylated H5 IgG (n=5); + H5 + sial = antigen + sialylated H5 IgG (n=5); neg. ctrl. = negative control.

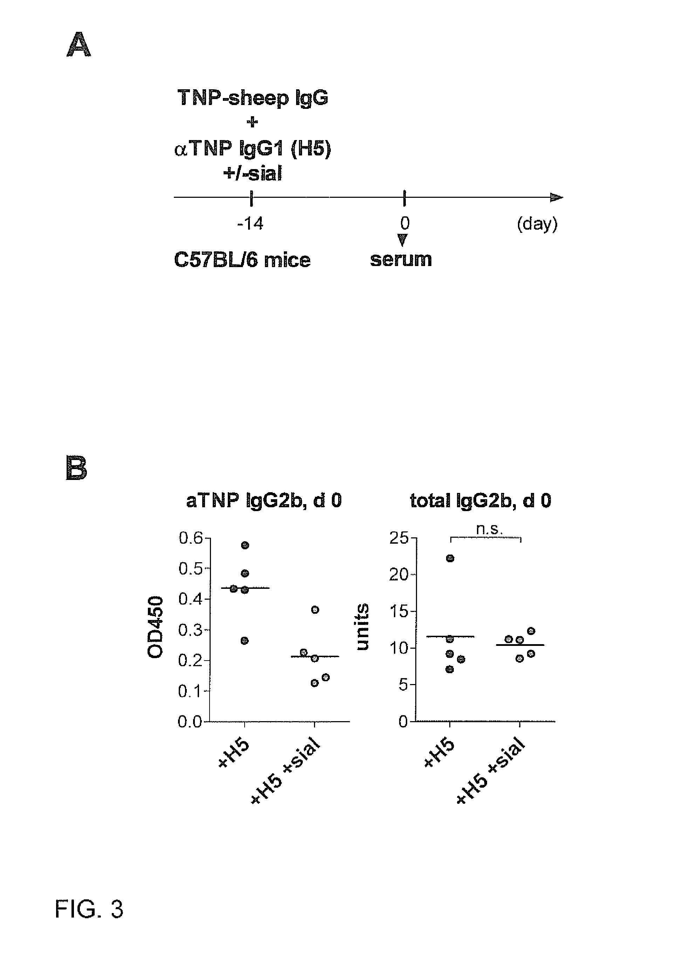

- FIG. 3: **(A)** shows a schematic representation of the treatment course; **(B)** summarizes the results at day 0, wherein titer of αTNP IgG2b antibodies and total IgG2b titers are given for animals treated either with immune complexes containing TNP-sheep IgG and non-sialylated H5 IgG (H5) or sialylated H5 IgG (H5 + sial).

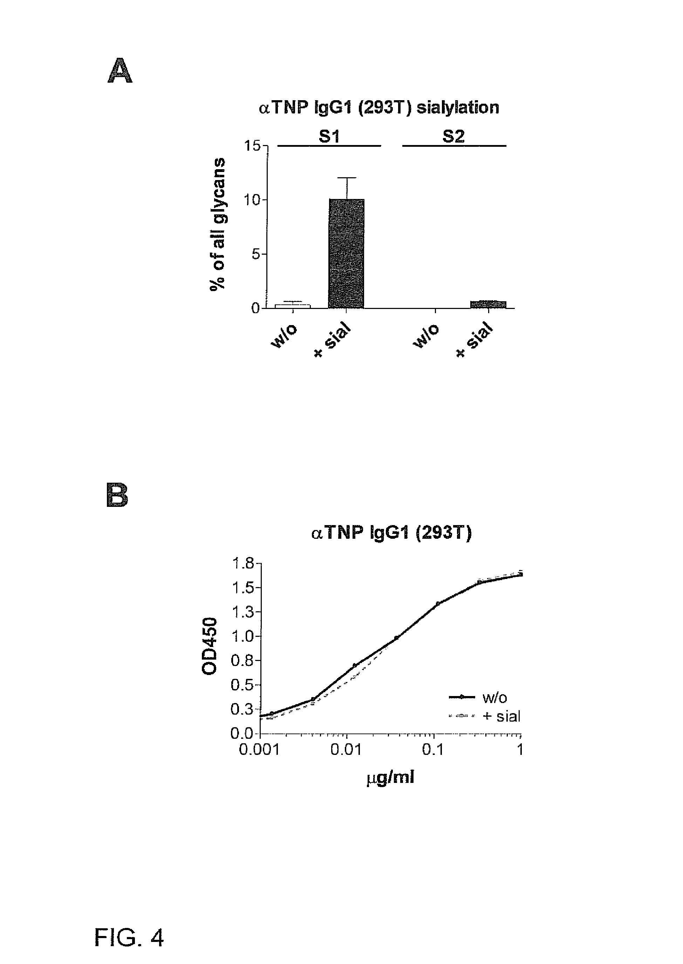

- FIG. 4: **(A)** shows results of sialylation (sial) of recombinant αTNP IgG in transformed 293T cells ; S1, one sialic acid; S2, two sialic acid; **(B)** shows that sialylation of αTNP IgG did not alter binding properties of recombinant αTNP IgG.

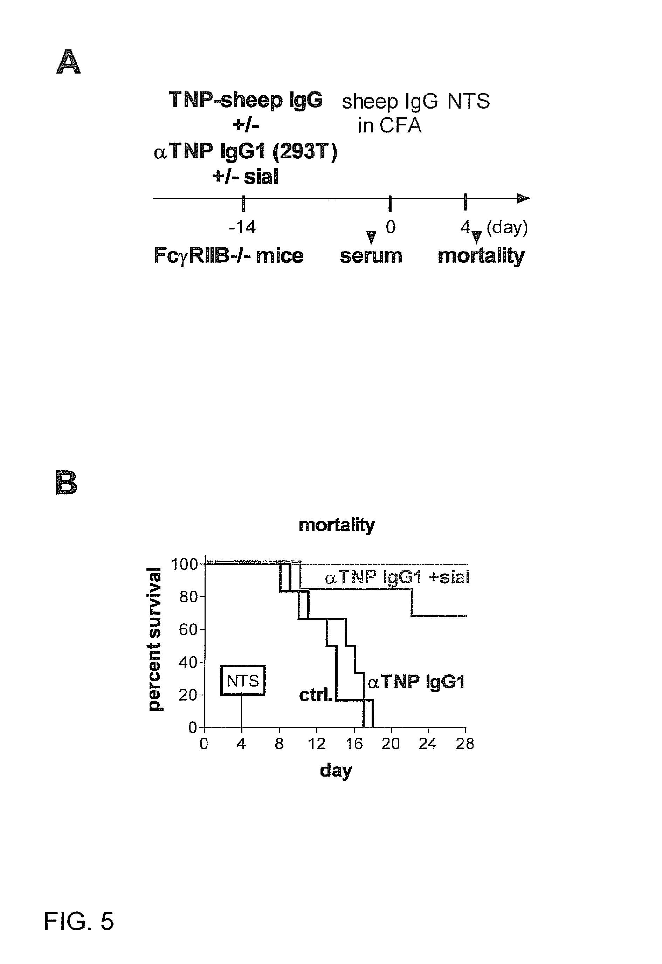

- FIG. 5: Immune complexes containing antigen plus sialylated recombinant IgGs protect from immune pathology (nephritis mouse model). **(A)** shows a schematic representation of the treatment course of the nephritis model; **(B)** summarizes the results of the nephrotoxicity model with regard to survival over time, wherein ctrl. = TNP-sheep IgG (antigen) without αTNP IgG (293T) (n=6); + αTNP IgG1 = antigen + non-sialylated IgG (n=6); αTNP IgG1 + sial = antigen + sialylated IgG (n=6).

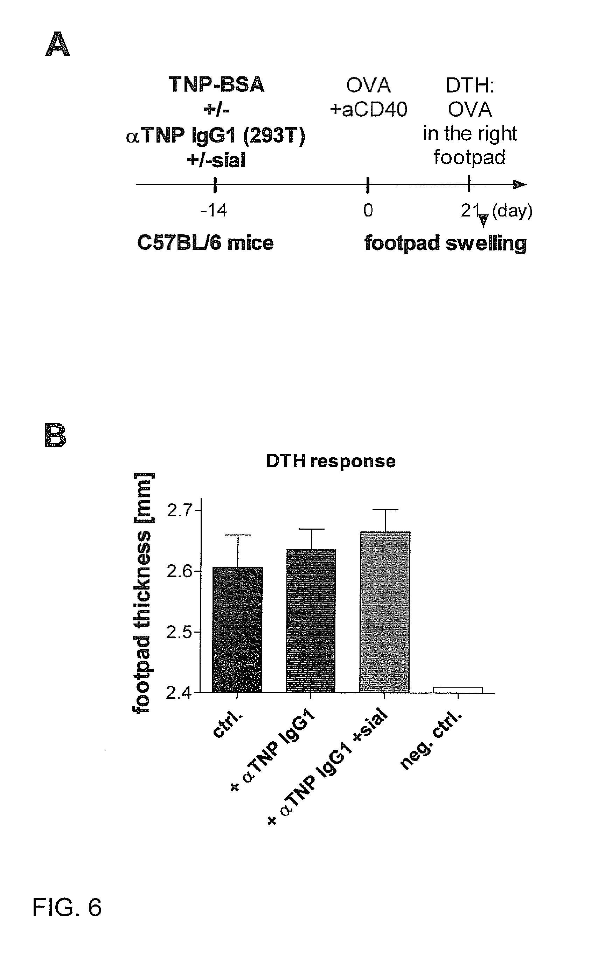

- FIG. 6: Immune complexes containing antigen plus sialylated recombinant IgGs do not inhibit immune pathology induced with an independent antigen **(A)** shows a schematic representation of the treatment course of a DTH model; **(B)** summarizes the results of the DTH model, wherein footpad thickness is given for the different treatments at day 3 after local DTH induction, ctrl. = TNP-BSA (antigen) without αTNP IgG (293T) antibody (n=7); + αTNP IgG1 = antigen + non-sialylated IgG (n=7); αTNP IgG1 + sial = antigen + sialylated IgG (n=7); neg. ctrl. = negative control.

### Example 1:

### Material and methods of example 1

### Mice.

C57BL/6 mice were purchased from Charles River Laboratories.
All mice were on a C57BL/6 background. Mice were bred and maintained in accordance with institutional guidelines. Female mice were analyzed exclusively.

### Reagents.

TNP-sheep IgG was an in house preparation.

### In vitro galactosylation and sialylation of IgG antibodies.

The murine αTNP IgG1hybridoma antibody (clone H5) (S. Wernersson et al., J. Immunol. 163, 618 (1999)) was purified from cell culture media with Protein-G sepharose and dialysed against PBS. *In vitro* glycosylation was performed in a two-step procedure as described previously (R. M. Anthony et al., Science 320, 373 (2008)). Briefly, 5mg H5 antibody was galactosylated by rotating incubation with 25mU β1,4-galactosyltransferase (Calbiochem) and 10µM UDPGalactose (Calbiochem) in 50mM MOPS, 20mM MnCl2, pH7.2 for 48h at 37°C. After buffer exchange to 50mM MES, 10mM MnCl2, pH6.0 IgG was sialylated by incubation with 25mU human α2,6-sialyltransferase (Calbiochem) and 0.5mM CMP-sialic acid (Calbiochem) for 48h at 37°C. The concentration of the differentially glycosylated murine αTNP IgG1 H5 antibodies was determined by BCA-Assay (Pierce), αTNP reactivity was verified by ELISA and N-glycosylation of IgG Fc fragments was characterized by MALDITOF mass spectrometry.

### Glycan analysis.

For MALDI-TOF mass spectrometry, IgG samples were digested with endoglycosidase S (EndoS) purified from *Streptococcus pyogenes* (M. Collin, A. Olsen, EMBO J. 20, 3046 (2001)). EndoS specifically cleaves the N-linked glycan at the Fc fragment exclusively from IgGs between the first and second GIcNAc (M. Collin, A. Olsen, EMBO J. 20, 3046 (2001)). Resulting N-glycans were purified by solid phase extraction using reversed-phase C18 and graphitized carbon columns (Alltech, Deerfield, IL). Samples were permethylated according to standard protocols and further investigated by MALDI-TOF mass spectrometry. Spectra were recorded on an Ultraflex III mass spectrometer (Bruker Daltonics) equipped with a Smartbeam laser. Calibration was performed on a glucose ladder and DHB was used as a matrix. Spectra were recorded in reflector positive ionization mode and mass spectra from 3000 laser shots were accumulated. Bar graphs indicate the mean value with standard error of the mean (SEM).

### Immune complex formation.

For immune complex formation 20µg TNP-sheep IgG and 80µg of the indicated αTNP IgG1 antibodies were incubated for 1 h at 37°C. Immune complexes were injected i.p.

### Delayed type hypersensitivity (DTH) response.

DTH response was induced by injection of 50µg sheep IgG in CFA or 200µg ovalbumin (Ova) plus 50µg αCD40 antibody at day 0 followed by injection of 12.5µg sheep IgG or Ova in montanide ISA 50V (Seppic) into the right footpad at the indicated day. PBS in montanide ISA 50V (Seppic) was injected into the left footpad as negative control. The intensity of the inflammatory reaction correlates with the footpad thickness, which was determined using an Oditest micrometer gauge in a blinded fashion (Kroeplin Laengenmesstechnik, Germany). Bar graphs indicate the mean value with standard error of the mean (SEM).

### ELISA.

For the detection of TNP specific IgG1 or IgG2b antibodies ELISA plates were coated with 100µl of 5µg/ml TNP-BSA in 0.05M Carbonate/Bicarbonate buffer pH9.6 (Sigma-Aldrich). Plates were blocked with 150µl PBS, 3% BSA, 1mM EDTA, 0.1 % gelatin and subsequently incubated with 1/100 diluted serum or different concentrations of purified antibody in PBS. IgG subclasses were detected with 100µl horseradish peroxidase-coupled goat anti-mouse IgG1or IgG2b specific secondary antibodies (Bethyl Laboratories). Symbols represent data from individual mice. Horizontal lines show mean values.

### Statistical analysis.

Statistical analyses were performed using Student's *t* test or Logrank test for survival curves: **P* < 0.05, ***P* < 0.01 and ****P* < 0.001.

### Results of example 1:

To determine whether immune complexes (ICs) consisting of antigen and antigen-specific sialylated IgG antibodies are sufficient to block antigen-specific pathogenic immune responses, we injected wild-type C57BL/6 mice with TNP-sheep IgG complexed with either non-sialylated (<1%) αTNP mouse IgG1 hybridoma H5 antibodies (H5) or *in vitro* sialylated (70%) H5 (H5 +sial) prior to DTH induction with sheep IgG in CFA **(****FIG. 1A** **and** **2A****).** Sialylation of H5 antibody did not influence TNP binding **(****FIG. 1B****)**. However, only ICs containing *in vitro* sialylated αTNP IgG1 (H5 +sial) antibodies (4mg/kg body weight) blocked a pathogenic delayed type hypersensitivity (DTH) immune reaction against sheep IgG **(****FIG. 2B****)** and blocked the development of T cell-dependent (TD) αTNP IgG2b antibodies but not total IgG2b antibodies **(****FIG. 3****).** Thus, ICs containing 4mg/kg body weight sialylated IgG (only 70% were sialylated) inhibited a later induced antigen-specific immune pathology.

### Example 2:

### Material and methods of example 2:

### Mice.

C57BL/6 mice were purchased from Charles River Laboratories. FcyRIIB-/- mice have been described previously (M. Ehlers, H. Fukuyama, T. L. McGaha, A. Aderem, J. V. Ravetch, J. Exp. Med. 203, 553 (2006)). All mice were on a C57BL/6 background. Mice were bred and maintained in accordance with institutional guidelines. Female mice were analyzed exclusively. Genotypes were determined by PCR on tail DNA as described previously (M. Ehlers, H. Fukuyama, T. L. McGaha, A. Aderem, J. V. Ravetch, J. Exp. Med. 203, 553 (2006)).

### Reagents.

TNP-coupled BSA was purchased from Biosearch Technology. TNP-sheep IgG was an in house preparation.

### Synthesis of sialylated αTNP IgG1 in human 293T cells.

The variable VDJ heavy chain region (NCBI X65772) and the complete kappa light chain (NCBI X65774) of the murine αTNP IgE hybridoma IgELa2 (ATCC-TIB142) (H. Kofler et al., Mol. Immunol. 29, 161 (1992)) were amplified by PCR from a generated cDNA (αTNP variable VDJ heavy chain part,
forward primer SEQ ID No. 6, 5'-tctaccggtgtacattccgaggtgcagcttcaggagtca,
reverse primer SEQ ID No 7, 5'-gcagggctagctgcagagacagtgaccagagtccc;
α TNP complete VJ kappa light chain,
forward primer SEQ ID No 8, 5' -gtcaccggtgtacattcagacattgtgatgtcacagtct,
reverse primer SEQ ID No. 9, 5'-ttattcggaagctttcaacactcattcctgttgaag).

To synthesize a murine αTNP IgG1 antibody the variable heavy chain region (*Agel-Nhel*) in combination with an amplified murine C57BL/6 IgG1 heavy constant region (*Nhe*I*-Bsi*WI)
(forward primer SEQ ID No 10, 5'-cctcgcgctagcacgacacccccatctgtctatccac,
reverse primer SEQ ID No 11, 5'-ttattcggcgtacgcgtcatttaccaggagagtgggag)
and the whole kappa light chain (*Age*I-*Hind*III) were cloned in recently described expression vectors (H. Wardemann et al., Science 301, 1374 (2003) and T. Tiller et al., J. Immunol. Methods 329, 112 (2008)). The leader sequences of the described expression vectors were used. The human ST6 β1,4-galactosamide α2,6-sialyltransferase 1 (ST6GAL1) (NCBI NM_003032) was amplified by PCR
(forward primer SEQ ID No 12, 5'-cctcgcctcgaggccaccatgattcacaccaacctgaag;
reverse primer SEQ ID No 13, 5'- tgcaggctgcagttagcagtgaatggtccggaa)
from human cDNA and cloned (*Xho*I-*Pst*I) into the expression vector pIRES2-EGFP (BD Biosciences Clontech) containing an IRES EGFP to control the transfection efficiency. Unsialylated and sialylated mouse αTNP IgG1 antibodies were produced by polyethylenimine-mediated co-transfection of 293T human embryonic kidney (HEK) cells with αTNP IgG1 IgH and IgL chain encoding plasmid DNA in the absence or presence of plasmid DNA encoding for the human ST6GAL1. 293T cells were cultured in DMEM high Glucose (GIBCO)
supplemented with 10% FCS and 1% Penicillin/Streptomycin (PS) in 145mm cell culture dishes. For transfection, cells were washed with PBS and cultured in 25ml DMEM high Glucose (GIBCO) containing 1% Primatone RL (MP Biomedicals) and 1 % PS. 10µg of IgH and IgL chain encoding plasmid DNA with or without 20µg of the ST6GAL1 expression plasmid in 3ml PBS were mixed thoroughly with 100µl (0.6mg/ml H₂O) polyethylenimine (PEI) (Sigma-Aldrich), incubated for 10 min at room temperature and poured to the cells. Supernatants were collected after 3 and 10 days of culture and the murine αTNP IgG1 was purified with Protein-G-Sepharose and dialysed against PBS. The concentration of the murine αTNP IgG1 was determined by BCA-Assay (Pierce) and SDS gel analysis, αTNP reactivity was verified by ELISA and N-glycosylation of IgG Fc fragments was characterized by MALDI-TOF mass spectrometry.

### Glycan analysis.

For MALDI-TOF mass spectrometry, IgG samples were digested with endoglycosidase S (EndoS) purified from *Streptococcus pyogenes* (M. Collin, A. Olsen, EMBO J. 20, 3046 (2001)). EndoS specifically cleaves the N-linked glycan at the Fc fragment exclusively from IgGs between the first and second GIcNAc (M. Collin, A. Olsen, EMBO J. 20, 3046 (2001)). Resulting N-glycans were purified by solid phase extraction using reversed-phase C18 and graphitized carbon columns (Alltech, Deerfield, IL). Samples were permethylated according to standard protocols and further investigated by MALDI-TOF mass spectrometry. Spectra were recorded on an Ultraflex III mass spectrometer (Bruker Daltonics) equipped with a Smartbeam laser. Calibration was performed on a glucose ladder and DHB was used as a matrix. Spectra were recorded in reflector positive ionization mode and mass spectra from 3000 laser shots were accumulated. Bar graphs indicate the mean value with standard error of the mean (SEM).

### Immune complex formation.

For immune complex formation 20µg TNP-sheep IgG or TNP-BSA and 80µg of the indicated αTNP IgG1 antibodies were incubated for 1 h at 37°C. Immune complexes were injected i.p.

### Delayed type hypersensitivity (DTH) response.

DTH response was induced by injection of 50µg sheep IgG or 200µg ovalbumin (Ova) plus 50µg αCD40 antibody at day 0 followed by injection of 12.5µg sheep IgG or Ova in montanide ISA 50V (Seppic) into the right footpad at the indicated day. PBS in montanide ISA 50V (Seppic) was injected into the left footpad as negative control. The intensity of the inflammatory reaction correlates with the footpad thickness, which was determined using an Oditest micrometer gauge in a blinded fashion (Kroeplin Laengenmesstechnik, Germany). Bar graphs indicate the mean value with standard error of the mean (SEM).

### Nephrotoxic nephritis model.

Nephritis was induced by injection of 50µg sheep IgG in CFA at day 0 followed by i.v. injection of sheep anti-glomerular basement membrane (GBM) nephrotoxic serum (NTS) 4 days later (M. P. Madaio, D. J. Salant, S. Adler, C. Darby, W. G. Couser, Kidney Int. 26, 397 (1984)).

### ELISA.

For the detection of TNP specific IgG1 antibodies ELISA plates were coated with 100µl of 5µg/ml TNP-BSA in 0.05M Carbonate/Bicarbonate buffer pH9.6 (Sigma-Aldrich). Plates were blocked with 150µl PBS, 3% BSA, 1mM EDTA, 0.1 % gelatin and subsequently incubated with 1/100 diluted serum or different concentrations of purified antibody in PBS. IgG subclasses were detected with 100µl horseradish peroxidase-coupled goat anti-mouse IgG1 specific secondary antibodies (Bethyl Laboratories).

### Statistical analysis.

Statistical analyses were performed using Student's *t* test or Logrank test for survival curves: **P* < 0.05, ***P* < 0.01 and ****P* < 0.001.

### Results of example 2:

The results of example 1 were confirmed by administration of ICs containing TNP-sheep IgG and
high (αTNP IgG1 +sial) or low (αTNP IgG1) sialylated αTNP IgG1 prior to the induction of nephritis with sheep IgG in CFA and NTS in FcyRIIB-/- mice **(****FIG. 4A** **and** **5A****).** Here, we used recombinant αTNP mouse IgG1, which was produced *in vitro* by transfection of human 293T cells with αTNP IgH and IgL chain encoding plasmids. Less than 1% of the resulting antibodies showed human sialic acid (Neu5Ac) residues (w/o) **(****FIG. 4A****).** Co-transfection of 293T cells with a plasmid mediating expression of human ST6 β1,4-galactosamide α2,6-sialyltransferase 1 (ST6GAL1) induced sialylation (Neu5Ac) up to 10% of IgG antibodies (+sial) whereas antibody-reactivity remained unaltered **(****FIG. 4A and B****).** Administration of TNP-sheep IgG with αTNP IgG1+sial antibodies (4mg/kg body weight) but not with αTNP IgG1 antibodies protected FcyRIIB-/- mice from sheep IgG-induced nephritis and mortality in an autoimmune nephritis model independent of FcgammaRIIB; Statistics: ctrl. versus αTNP IgG1 +sial, *p=0.010*; αTNP IgG1 versus αTNP IgG1 +sial, *p=0.007.* **(****FIG. 5B****).** Thus, ICs containing 4mg/kg body weight sialylated IgG (only 10% were sialylated) also inhibited a later induced antigen-specific nephritis. Administration of immune complexes consisting of TNP-BSA and αTNP IgG1 +sial antibodies (4mg/kg body weight) at day -14 did not inhibit a pathogenic immune reaction induced with chicken ovalbumin plus αCD40 at day 0 demonstrating that the sialylated IgGs acted in this concentration only antigen-specifically **(****FIG. 6****).**

**Table 1:**

| **autoimmune diseases** | | | |
|---|---|---|---|
| **Antigen** | **UniProt ID** | **NCBI Accession** | **autoimmune disease** |
| | | **Nr.** | |
| red blood cell antigens (e.g. Kell) | P23276 | NM_000420 | Autoimmune hemolytic anemia |
| Soluble Liver Antigen | Q9HD40, | NM_016955 | Autoimmune Hepatitis |
| (SLA/LP), | | NM_153825 | |
| | A1A4F3, | NM_153825 | |
| | Q0D2P3 | BC023539 | |
| liver/kidney microsomal | | | |
| antigen (LKM-1) | | | |
| asialoglycoprotein receptor | P07306, | NM_001671 | Autoimmune Hepatitis |
| (ASGR1, | | | |
| ASGR2) | P07307 | NM_001181 | |
| | | NM_080912 | |
| | | NM_080913 | |
| | | NM_080914 | |
| Liver kidney microsomal | Q6NXU8 | NM_001025161 | Autoimmune Hepatitis |
| antigen (LKM-1) | | | Hepatitis Type C Virus |
| e.g. cytochrome P450 2D6 (CYP2D6) | Q6NWU0 | NM_000106 | |
| Actin | P68032, | NM_005159 | Autoimmune Hepatitis |
| | P68133, | NM_001100 | |
| | P62736, | NM_001141945 | |
| | | NM_001613 | |
| | P63267 | NM_001615 | |
| transglutaminase | P22735, | NM_000359 | coeliac disease |
| | P21980, | NM_004613 | |
| | | NM_198951 | |
| | O43548, | NM_004245 | |
| | | NM_201631 | |
| | P49221, | NM_003241 | |
| | Q08188 | NM_003245 | |
| Glutamic Acid | Q99259 | NM_000817 | Diabetes mellitus type 1 |
| Decarboxylase (GAD) | | NM_013445 | |
| | Q05329 | NM_000818 | |
| | | NM_001134366 | |
| ROS¹ modified GAD65 | | | Diabetes mellitus type 1 |
| Insulinoma Associated Protein-2 | Q96T92 | NM_032594 | Diabetes mellitus type 1 |
| insulin | P01308 | NM_000207 | Diabetes mellitus type 1 |
| islet cell antigens | Q05084 | NM_001136020 | Diabetes mellitus type 1 |
| | | NM_004968 | |
| | | NM_022307 | |
| | | | |
| Glomerular Basement | Q01955 | NM_001130105 | goodpasture's disease |
| Membrane antigens | | NM_031361 | |
| (e.g. alpha-3 chain of Type | | NM_005713 | |
| IV collagen) | | NM_000091 | |
| | | NM_031362 | |
| | | NM_031366 | |
| | | NM_031365 | |
| TSH receptor | P16473 | NM_001142626 | Graves'disease |
| | Q8TB90 | NM_001018036 | |
| | | NM_000369 | |
| thyroglobulin | P01266 | NM_003235 | Graves'disease |
| Ganglioside Antigens | | | Guillain-Barré syndrome |
| (GD3, | | | |
| GM1, | | | |
| GM2, | P17900 | NM_000405 | |
| GQ3, | | | |
| Anti-GQ1b | | | |
| and GT1) | | | |
| thyroid peroxidase | P07202 | NM_175719 | Hashimoto's thyroiditis and |
| | | NM_175721 | Ord's thyroiditis and other |
| | | NM_175722 | forms of thyroiditis |
| | | NM_000547 | |
| thyroglobulin | | | |
| TSH receptors | | | |
| further antigens of the | | | |
| thyroid gland | | | |
| platelet membrane antigens (GP2B | P08514 | NM_000419 | Idiopathic thrombocytopenic purpura |
| GP3A | P05106 | NM_000212 | |
| GP1BB | P13224 | NM_000407 | |
| GP1BA | P07359 | NM_000173 | |
| GP9) | P14770 | NM_000174 | |
| myelin basic protein | P02686 | NM_001025081 | Multiple sclerosis |
| | | NM_001025090 | |
| | | NM_001025092 | |
| | | NM_001025100 | |
| | | NM_001025101 | |
| | | NM_002385 | |
| myelin oligodendrocyte | Q16653 | NM_206813 | Multiple sclerosis |
| glycoprotein | | | |
| proteolipid protein | P60201 | NM_000533 | Multiple sclerosis |
| | | NM_001128834 | |
| | | NM_199478 | |
| acetylcholine receptor | P02708, | NM_001039523 | Myasthenia Gravis |
| | P17787, | NM_000748 | |
| | Q04844, | NM_000080 | |
| | Q07001, | NM_000751 | |
| | P07510 | NM_005199 | |
| muscle specific kinase | 015146 | NM_005592 | Myasthenia Gravis |
| citrullinated protein antigen | P20930 | NM_002016 | Rheumatoid arthritis |
| (e.g. citrullinated filaggrin, | | | |
| MBP, histone, collagen, | | | |
| fibrin, fibrinogen, vimentin) | | | |
| collagen, type II, alpha 1 | P02458 | BC007252, | Rheumatoid arthritis |
| (COL2A1) | | BC116449, | |
| | | BT007205, | |
| | | NM_001844, | |
| | | NM_033150, | |
| | | NG_008072, | |
| | | AH002813; | |
| | | X13783 | |
| reactive oxygen species | | | Rheumatoid arthritis |
| modified (ROS) collagen-2 | | | Alzheimers disease |
| dsDNA, ssDNA, ssRNA | | | systemic lupus |
| | | | erythematosus |
| Smith antigens (SmB, | P14678, | NM_003091 | systemic lupus |
| SmD1, SmD2, SmD3, | | NM_198216 | erythematosus |
| SmE, SmF, SmG) | | | |
| | P62314, | NM_006938 | |
| | P62318, | NM_004175 | |
| | P62316, | NM_004597 | |
| | | NM_177542 | |
| | P62304, | NM_003094 | |
| | P62306, | NM_003095 | |
| | P62308 | NM_003096 | |
| Histone (H1, | P07305 | NM_005318 | systemic lupus |
| | Q02539 | NM_005325 | erythematosus |
| | P 16403 | NM_005319 | |
| | P 16402 | NM_005320 | |
| | P 10412 | NM_005321 | |
| | P 16401 | NM_005322 | |
| | P22492 | NM_005323 | |
| | Q4VB24, | | |
| H2A, | A3KPC7, | NM_080596 | |
| H2B, | A8K110, | NM_003528 | |
| H3, H4) | Q5TEC6, | | |
| | Q0VAS5 | | |
| SS-A (Ro) | P10155, | NM_001042369 | systemic lupus |
| | | | erythematosus, Sjoegrens |
| | P19474 | NM_003141 | syndrome |
| SS-B (La) | P05455 | NM_003142 | systemic lupus |
| | | | erythematosus, |
| | | | Sjoegren's syndrome |
| PDGF receptor | P16234, | NM_006206 | systemic sclerosis |
| | P09619 | NM_002609 | |
| Transmembrane type XVII collagen (BP180, BPAG2, BP230) | | NM_000494, NG_007069 | bullous pemphigoid |
| LAD-1 (processed | | NM_005558, | Linear IgA disease (LAD) |
| extracellular form of type | | NM_000494, | |
| XVII collagen) | | NG_007069 | |
| Neutrophil cytoplasmic | | | Churg-Strauss syndrome, |
| antigens | | | Wegener's |
| | | | granulomatosis, systemic |
| | | | lupus erythematosus |
| Gliadin (gluten protein in | | | Coeliac disease |
| wheat) leads to crossreactivity with the bowel tissue | | | |
| 21-hydroxylase | | | Addison's disease induced by adrenal destruction, autoimmune polyendocrine syndrome |
| Phospholipids, e.g. cardiolipin and β2 glycoprotein I | | | Anti-phospholipid syndrome |

| **prevention of immunization** | | | |
|---|---|---|---|
| **Antigen** | **UniProt ID** | **NCBI Accession Nr.** | **prevention of immunization** |
| Human Rhesus D antigen | Q02161 | NM_001127691 NM_016124 NG_007494, X63094, X63097, AW805821, X63097 | Anti-D prophylaxis |

| **animal allergy** | | | |
|---|---|---|---|
| **Antigen** | **UniProt ID** | **NCBI Accession** | **animal allergy** |
| | | **Nr.** | |
| Fel d1 | P30438, | NM_001048153 | cat allergy |
| | P30440 | NM_001048154 | |
| Can f1 | 018873 | NM_001003190 | dog allergy |
| house dust mite antigens | Q8MWR5, | AF409110 | house dust mite allergy |
| (e.g. Der p serine | | | |
| proteases, | P08176, | U 11695 | |
| Derp1, | | | |
| Derf1, | P16311, | X65196 | |
| Derf2, | Q00855, | D10447 | |
| | | D10448 | |
| | | D10449 | |
| | | S70378 | |
| Derp2, | P49278, | AF276239 | |
| Derp5) | P14004 | S76337 | |
| | | S76340 | |
| | | X17699 | |
| bee sting venom antigens | | | bee sting allergy |
| Phospholipase A2 | P00630 | NM_001011614 | Apis mellifera (Honeybee) |
| Hyaluronidase | Q08169 | NM_001011619 | |
| Melittin (Allergen Api m III) | P01501 | NM_001011607 | |
| Allergen Api m 6 | P83563 | NM_001040270 | |
| | | NM_001040268 | |
| | | DQ384990 | |
| | | DQ384991 | |
| wasp sting venom antigens | | | wasp sting allergy |
| Venom allergen 5 | Q05110 | M98858 | Vespula vulgaris (Yellow |
| | | | jacket) (Wasp) |
| Hyaluronidase A | P49370 | L43562 | |
| Hyaluronidase B | Q5D7H4 | AY845866 AJ920395 | |
| Phospholipase A1 | P49369 | L43561 | |

| **pollen allergy** | | | |
|---|---|---|---|
| **Antigen** | **UniProt ID** | **NCBI Accession Nr.** | **pollen allergy** |
| Kentucky bluegrass pollen | P22284 | M38342 | Kentucky bluegrass pollen |
| allergens | P22285 | M38343 | allergy |
| | P22286 | M38344 | |
| Orchard grass pollen | P93124 | U25343 | Orchard grass pollen |
| allergens | Q41183 | AJ131334 | allergy |
| | P82946 | AY241677 | |
| | | AY241676 | |
| Sweet vernal grass pollen | Q7M1Y0 | | Sweet vernal grass pollen |
| allergens | Q7M1X6 | | allergy |
| Johnson grass pollen | | | Johnson grass pollen |
| antigens | | | allergy |
| Bermuda grass pollen | P94092 | X91256 | Bermuda grass pollen |
| antigens | | U75585 | allergy |
| Polcalcin Cyn d 7 | | | |
| Major pollen allergen Cyn | 004701 | S83343 | |
| d 1 | | | |
| Profilin | 004725 | Y08390 | |
| | | Y08389 | |
| ryegrass pollen antigens | P14946 | M57474 | ryegrass pollen allergy |
| | | M57476 | |
| | Q40237 | L13083 | |
| | P14947 | | |
| | P14948 | | |
| | Q40240 | M59163 | |
| | Q7M1X5 | | |
| timothy-grass pollen | P43214 | | timothy-grass pollen |
| antigens | P43213 | | allergy |
| (Phleum pratense) | Q40963 | | |
| | 082040 | | |
| | P35079 | | |
| | 024650 | | |
| | 024282 | | |
| | Q40962 | | |
| | P43215 | | |
| | Q8H6L7 | | |
| | 065868 | | |
| ragweed pollen antigens | P10414 | | ragweed pollen allergy |
| (Ambrosia trifida | P27759 | | |
| Ambrosia artemisiifolia) | P27760 | | |
| | P27762 | | |
| | P27761 | | |
| | P28744 | | |
| | P02878 | | |
| | 004004 | | |
| | P00304 | | |
| | P43174 | | |
| | P43175 | | |
| | Q64LH2 | | |
| | Q64LH0 | | |
| | Q64LH1 | | |
| plantago pollen antigens | P82242 | AJ313166 | plantago pollen allergy |
| | | AJ313167 | |
| | | AJ313168 | |
| nettle pollen antigens | | | nettle pollen allergy |
| artemisia vulgaris pollen | Q7M1G9 | | artemisia vulgaris pollen |
| antigens | Q84ZX5 | | allergy |
| | Q8H2C9 | | |
| | Q8H2C8 | | |
| | P0C088 | | |
| chenopodium album pollen | Q84V36 | | chenopodium album |
| antigens | Q84V37 | | pollen allergy |
| | Q8LGR0 | | |
| sorrel pollen antigens | | | sorrel pollen allergy |
| birch pollen antigens (e.g. | P15494 | | birch pollen allergy |
| Bet v1a, Bet v2,Bet v3, Bet | P25816 | | |
| v4) | Q39419 | | |
| | P43185 | | |
| | P45431 | | |
| | P43176 | | |
| | P43178 | | |
| | P43180 | | |
| | P43183 | | |
| | P43184 | | |
| | P43177 | | |
| | P43179 | | |
| | P43186 | | |
| | P81531 | | |
| | P43187 | | |
| alder pollen antigens | 081701 | | alder pollen allergy |
| | P38948 | | |
| hazel pollen antigens | Q08407 | X70999 | hazel pollen allergy |
| | | X71000 | |
| | | X70997 | |
| | | X70998 | |
| hornbeam pollen antigens | P38949 | | hornbeam pollen allergy |
| | P38950 | | |
| aesculus pollen antigens | | | aesculus pollen allergy |
| willow pollen antigens | | | willow pollen allergy |
| poplar pollen antigens | | | poplar pollen allergy |
| platanus pollen antigens | Q6H9K0 | | platanus pollen allergy |
| | P85814 | | |
| | Q8GT41 | | |
| tilia pollen antigens | | | tilia pollen allergy |
| olea pollen antigens | P19963 | | olea pollen allergy |
| | 024172 | | |
| | Q9M7R0 | | |
| | 081092 | | |
| | 024169 | | |
| | 024170 | | |
| | 024171 | | |
| | P80740 | | |
| | P81430 | | |
| | P80741 | | |
| Ashe juniper pollen | P81294 | | Ashe juniper pollen allergy |
| antigens | P81295 | | |
| | Q9FY19 | | |
| | Q9LLT1 | | |
| | P81826 | | |
| elm tree pollen antigens | | | elm tree pollen allergy |
| maple pollen antigens | | | maple pollen allergy |
| yew pollen antigens | | | yew pollen allergy |
| oak tree pollen antigens | P85126 | | oak tree pollen allergy |
| ash tree pollen antigens | Q6U740 | | ash tree pollen allergy |
| | Q7XAV4 | | |
| | Q5EXJ6 | | |
| rape pollen antigens | P80208 | | rape pollen allergy |
| | P69196 | | |
| | P69198 | | |
| rye pollen antigens | | | rye pollen allergy |
| Maize pollen antigens | Q07154 | NM_001111739 | |

| **food allergy** | | | |
|---|---|---|---|
| **Antigen** | **UniProt ID** | **NCBI Accession** | **food allergy** |
| | | **Nr.** | |
| peanut antigens (e.g. Ara | Q6PSU2 | | peanut allergy |
| h1, Ara h2) | Q647G9 | | |
| | P43238 | | |
| | P43237 | | |
| | Q9SQ19 | | |
| walnut antigens | P93198 | | walnut allergy |
| | Q2TPW5 | | |
| | Q9SEW4 | | |
| almond antigens | Q8GSL5 | | almond allergy |
| | P82944 | | |
| | P82952 | | |
| cashew antigens | Q8GZP6 | | cashew allergy |
| | Q8L5L6 | | |
| | Q8H2B8 | | |
| | Q8L5L5 | | |
| Hazelnut antigens | Q9FPK2 | | |
| | Q9FPK4 | | |
| | Q9FPK3 | | |
| | Q9SWR4 | | |
| | Q8W1C2 | | |
| | Q9AXH5 | | |
| | Q9AXH4 | | |
| | Q8S4P9 | | |
| | Q84T91 | | |
| Pistachio antigens | B2KN55 | | |
| | B4X640 | | |
| Wheat gliadin | P21292, | M11075 | coeliac disease |
| | P08453, | M11076 | |
| | P04721, | M11074 | |
| | P04722, | K03075 | |
| | P04723, | M11073 | |
| | P04724, | K03074 | |
| | P04725 | M10092 | |

| **drug allergy** | | | |
|---|---|---|---|
| **Antigen** | **UniProt ID** | **NCBI Accession Nr.** | **drug allergy** |
| penicillin (beta-lactam antibiotics) | | | penicillin allergy |
| sulfonamides | | | sulfonamides allergy |

## Claims

1. Method of producing an sialylated antibody specific for a human autoimmune antigen, allergen or human Rhesus factor D antigen, comprising an Fc-portion of IgG type, and exhibiting a sialic acid residue at the Fc-portion, comprising the following steps:
a) isolation of B cells from an individual suffering from an autoimmune disease, allergy or exhibiting an immune response to human Rhesus factor D antigen and individualisation of isolated B cells;
b) optionally, cloning of selected B cells;
c) optionally, selection of B cell clone that provides native antibody specific for human Rhesus factor D antigen or an antigen associated with autoimmune disease or allergy of donor of B cells;
d) isolation of the whole or part of the nucleic acid coding sequence encoding the native antibody provided by an individualized B cell or a cell clone originating from an individualized B cell and being specific for human Rhesus factor D antigen or an antigen associated with autoimmune disease or allergy of donor of B cells;
e) use of isolated native antibody nucleic acid coding sequence as a whole or in part to provide a nucleic acid coding sequence encoding an antibody with specificity for the same antigen as the respective native antibody and exhibiting an Fc-portion of IgG type, preferably of human IgG type;
f) expression of antibody encoded by the nucleic acid coding sequence of step e); and
g) addition of sialic acid residue to the Fc-portion of the expressed antibody.

2. Method of claim 1, wherein the sialic acid residue is added by the host used for expression of the antibody or is added to the expressed antibody in vitro.

3. Method of anyone of claims 1 to 2, wherein expression is performed in a host that expresses β1,4-galactosyltransferase and /or α2,6-sialyltransferase, preferably human β1,4-galactosyltransferase and/or human α2,6-sialyltransferase.

4. Method of producing a sialylated antibody specific for a human autoimmune antigen, allergen or human Rhesus factor D antigen, comprising an Fc-portion of IgG type, and exhibiting a sialic acid residue at the Fc-portion, comprising the following steps:
i) providing blood sample from an individual suffering from an autoimmune disease, allergy or exhibiting an immune response to human Rhesus factor D antigen or an autoimmune antigen or allergen;
ii) isolation of IgG antibody specific for human Rhesus factor D antigen or an autoimmune antigen or allergen from blood sample; and
iii) addition of sialic acid residue to the Fc-portion of the isolated IgG antibody.

5. Method of anyone of claims 1 to 4, wherein the sialic acid residue is added to amino acid Asn²⁹⁷ or a homologous position of the Fc-portion.

6. Method of anyone of claims 1 to 5, wherein the antigen is selected from table 1.

7. Sialylated antibody obtainable by a method of anyone of claims 1 to 6.

8. Sialylated isolated antibody specific for an antigen selected from autoimmune antigens, allergens or Rhesus factor D antigen, comprising an Fc-portion of IgG type, and exhibiting a sialic acid residue at the Fc-portion.

9. Sialylated antibody according to claims 7 or 8, wherein the antibody exhibits a binding affinity of ≤ 10⁻⁶M for the antigen or allergen.

10. Sialylated antibody according to anyone of claims 7 to 9, wherein the sialic acid residue is positioned at amino acid Asn²⁹⁷ or a homologous position of the Fc-portion.

11. Antibody according to anyone of claims 7 to 10, wherein the antigen is selected from table 1.

12. Immune complex comprising an antibody according to one of the claims 7 to 11 specifically bound to its antigen.

13. Pharmaceutical composition comprising an antibody according to one of claims 7 to 11 or an immune complex of claim 12 and a pharmaceutically acceptable excipient.

14. Kit comprising an antibody according to one of claims 7 to 11, an immune complex of claim 12, a pharmaceutical composition of claim 13.

15. Antibody according to anyone of claims 7 to 11, immune complex of claim 12, pharmaceutical composition of claim 13 or a kit of claim 14 for treatment and/or prophylaxis of autoimmune disease, allergy or Rhesus factor D reactivity.


SEQUENCE LISTING

<110>  Deutsches Rheuma-Forschungszentrum Berlin

<120>  Sialylated antigen-specific antibodies for treatment or
       prophylaxis of unwanted immune reactions and methods of producing
        them

<130>  P627209EP

<160>  18

<170>  PatentIn version 3.3

<210>  1
<211>  98
<212>  PRT
<213>  human


<220>
<221>  misc_feature
<223>  IgG1 CH1 region

<400>  1

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Gln Thr
65                  70                  75                  80


Tyr Ile Cys Asn Val Asn His Lys Pro Ser Asn Thr Lys Val Asp Lys
                85                  90                  95


Lys Val



<210>  2
<211>  15
<212>  PRT
<213>  human


<220>
<221>  misc_feature
<223>  IgG1 Hinge region

<400>  2

Glu Pro Lys Ser Cys Asp Lys Thr His Thr Cys Pro Pro Cys Pro
1               5                   10                  15

<210>  3
<211>  110
<212>  PRT
<213>  human

<220>
<221>  misc_feature
<223>  IgG1 CH2 region

<400>  3

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110

<210>  4
<211>  107
<212>  PRT
<213>  human

<220>
<221>  misc_feature
<223>  IgG1 CH3 region

<400>  4

Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

<210>  5
<211>  11
<212>  PRT
<213>  human

<220>
<221>  misc_feature
<223>  Fc-portion N-glycosilation motif

<400>  5

Arg Glu Glu Gln Tyr Asn Ser Thr Tyr Arg Val
1               5                   10

<210>  6
<211>  39
<212>  DNA
<213>  artificial

<220>
<223>  primer

<400>  6
tctaccggtg tacattccga ggtgcagctt caggagtca                              39

<210>  7
<211>  35
<212>  DNA
<213>  artificial

<220>
<223>  primer

<400>  7
gcagggctag ctgcagagac agtgaccaga gtccc                                  35

<210>  8
<211>  39
<212>  DNA
<213>  artificial

<220>
<223>  primer

<400>  8
gtcaccggtg tacattcaga cattgtgatg tcacagtct                              39

<210>  9
<211>  36

<212>  DNA
<213>  artificial

<220>
<223>  primer

<400>  9
ttattcggaa gctttcaaca ctcattcctg ttgaag                                36


<210>  10
<211>  37
<212>  DNA
<213>  artificial

<220>
<223>  primer

<400>  10
cctcgcgcta gcacgacacc cccatctgtc tatccac                               37


<210>  11
<211>  38
<212>  DNA
<213>  artificial

<220>
<223>  primer

<400>  11
ttattcggcg tacgcgtcat ttaccaggag agtgggag                              38


<210>  12
<211>  39
<212>  DNA
<213>  artificial

<220>
<223>  primer

<400>  12
cctcgcctcg aggccaccat gattcacacc aacctgaag                             39


<210>  13
<211>  33
<212>  DNA
<213>  artificial

<220>
<223>  primer

<400>  13
tgcaggctgc agttagcagt gaatggtccg gaa                                   33


<210>  14
<211>  326
<212>  PRT
<213>  human


<220>
<221>  misc_feature
<223>  human  IgG1 CH1, Hinge, CH2 and CH3 sequence

<400> 14

Ser Thr Lys Gly Pro Ser Val Phe Pro Leu Ala Pro Ser Ser Lys Ser
1               5                   10                  15

Thr Ser Gly Gly Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Ser Leu Gly Thr Gln Thr Tyr
65                  70                  75                  80

Ile Cys Asn Val Asn His Lys Pro Ser Asn Thr Lys Val Asp Lys Lys
                85                  90                  95

Val Pro Lys Ser Cys Asp Lys Thr His Thr Cys Pro Pro Cys Pro Pro
            100                 105                 110

Glu Leu Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser His Glu Asp Pro Glu Val Lys Phe Asn Trp Tyr Val Asp
145                 150                 155                 160

Gly Val Glu Val His Asn Ala Lys Thr Lys Pro Arg Glu Glu Gln Tyr
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Ala Leu
        195                 200                 205

Pro Ala Pro Ile Glu Lys Thr Ile Ser Lys Ala Lys Gln Pro Arg Glu
    210                 215                 220

Pro Gln Val Tyr Thr Leu Pro Pro Ser Arg Asp Glu Leu Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Val Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  15
<211>  322
<212>  PRT
<213>  human

<220>
<221>  misc_feature
<223>  human  IgG2 CH1, Hinge, CH2 and CH3 sequence

<400>  15

Ser Thr Lys Gly Pro Ser Val Phe Pro Leu Ala Pro Cys Ser Arg Ser
1               5                   10                  15

Thr Ser Glu Ser Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Asn Phe Gly Thr Gln Thr Tyr
65                  70                  75                  80

Thr Cys Asn Val Asp His Lys Pro Ser Asn Thr Lys Val Asp Lys Thr
                85                  90                  95

Val Arg Lys Cys Cys Val Glu Cys Pro Pro Cys Pro Pro Pro Val Ala
            100                 105                 110

Gly Pro Ser Val Phe Leu Phe Pro Pro Lys Pro Lys Asp Thr Leu Met
        115                 120                 125

Ile Ser Arg Thr Pro Glu Val Thr Cys Val Val Val Asp Val Ser His
    130                 135                 140

Glu Asp Pro Glu Val Gln Phe Asn Trp Tyr Val Asp Gly Val Glu Val
145                 150                 155                 160

His Asn Ala Lys Thr Lys Pro Arg Glu Glu Gln Phe Asn Ser Thr Phe
                165                 170                 175

Arg Val Val Ser Val Leu Thr Val Val His Gln Asp Trp Leu Asn Gly
            180                 185                 190

Lys Glu Tyr Lys Cys Lys Val Ser Asn Lys Gly Leu Pro Ala Pro Ile
        195                 200                 205

Glu Lys Thr Ile Ser Lys Thr Lys Gln Pro Arg Glu Pro Gln Val Tyr
    210                 215                 220

Thr Leu Pro Pro Ser Arg Glu Glu Met Thr Lys Asn Gln Val Ser Leu
225                 230                 235                 240

Thr Cys Leu Val Lys Gly Phe Tyr Pro Ser Asp Ile Ala Val Glu Trp
                245                 250                 255

Glu Ser Asn Gly Gln Pro Glu Asn Asn Tyr Lys Thr Thr Pro Pro Met
            260                 265                 270

Leu Asp Ser Asp Gly Ser Phe Phe Leu Tyr Ser Lys Leu Thr Val Asp
        275                 280                 285

Lys Ser Arg Trp Gln Gln Gly Asn Val Phe Ser Cys Ser Val Met His
    290                 295                 300

Glu Ala Leu His Asn His Tyr Thr Gln Lys Ser Leu Ser Leu Ser Pro
305                 310                 315                 320

Gly Lys

<210>  16
<211>  370
<212>  PRT
<213>  human

<220>
<221>  misc_feature
<223>  human  IgG3 CH1, Hinge, CH2 and CH3 sequence

<400>  16

Ser Thr Lys Gly Pro Ser Val Phe Pro Leu Ala Pro Cys Ser Arg Ser
1               5                   10                  15

Thr Ser Gly Gly Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Ser Leu Gly Thr Gln Thr Tyr
65                  70                  75                  80

Thr Cys Asn Val Asn His Lys Pro Ser Asn Thr Lys Val Asp Lys Arg
                85                  90                  95

Val Leu Lys Thr Pro Leu Gly Asp Thr Thr His Thr Cys Pro Arg Cys
            100                 105                 110

Pro Pro Lys Ser Cys Asp Thr Pro Pro Pro Cys Pro Arg Cys Pro Pro
        115                 120                 125

Lys Ser Cys Asp Thr Pro Pro Pro Cys Pro Arg Cys Pro Pro Lys Ser
    130                 135                 140

Cys Asp Thr Pro Pro Pro Cys Pro Arg Cys Pro Pro Glu Leu Leu Gly
145                 150                 155                 160

Gly Pro Ser Val Phe Leu Phe Pro Pro Lys Pro Lys Asp Thr Leu Met
                165                 170                 175

Ile Ser Arg Thr Pro Glu Val Thr Cys Val Val Val Asp Val Ser His
            180                 185                 190

Glu Asp Pro Glu Val Gln Phe Lys Trp Tyr Val Asp Gly Val Glu Val
        195                 200                 205

His Asn Ala Lys Thr Lys Pro Arg Glu Glu Gln Tyr Asn Ser Thr Phe
    210                 215                 220

Arg Val Val Ser Val Leu Thr Val Leu His Gln Asp Trp Leu Asn Gly
225                 230                 235                 240

Lys Glu Tyr Lys Cys Lys Val Ser Asn Lys Ala Leu Pro Ala Pro Ile
                245                 250                 255

Glu Lys Thr Ile Ser Lys Thr Lys Gln Pro Arg Glu Pro Gln Val Tyr
            260                 265                 270

Thr Leu Pro Pro Ser Arg Glu Glu Met Thr Lys Asn Gln Val Ser Leu
        275                 280                 285

Thr Cys Leu Val Lys Gly Phe Tyr Pro Ser Asp Ile Ala Val Glu Trp
    290                 295                 300

Glu Ser Ser Gly Gln Pro Glu Asn Asn Tyr Asn Thr Thr Pro Pro Met
305                 310                 315                 320

Leu Asp Ser Asp Gly Ser Phe Phe Leu Tyr Ser Lys Leu Thr Val Asp
                325                 330                 335

Lys Ser Arg Trp Gln Gln Gly Asn Ile Phe Ser Cys Ser Val Met His
            340                 345                 350

Glu Ala Leu His Asn Arg Phe Thr Gln Lys Ser Leu Ser Leu Ser Pro
        355                 360                 365

Gly Lys
    370

<210> 17
<211> 323
<212> PRT
<213> human

<220>
<221> misc_feature
<223> human IgG4 CH1, Hinge, CH2 and CH3 sequence

<400> 17

Ser Thr Lys Gly Pro Ser Val Phe Pro Leu Ala Pro Cys Ser Arg Ser
1               5                   10                  15

Thr Ser Glu Ser Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Ser Leu Gly Thr Lys Thr Tyr
65                  70                  75                  80

Thr Cys Asn Val Asp His Lys Pro Ser Asn Thr Lys Val Asp Lys Arg
                85                  90                  95

Val Ser Lys Tyr Gly Pro Pro Cys Pro Ser Cys Pro Pro Glu Phe Leu
            100                 105                 110

Gly Gly Pro Ser Val Phe Leu Phe Pro Pro Lys Pro Lys Asp Thr Leu
        115                 120                 125

Met Ile Ser Arg Thr Pro Glu Val Thr Cys Val Val Val Asp Val Ser
    130                 135                 140

Gln Glu Asp Pro Glu Val Gln Phe Asn Trp Tyr Val Asp Gly Val Glu
145                 150                 155                 160

Val His Asn Ala Lys Thr Lys Pro Arg Glu Glu Gln Phe Asn Ser Thr
                165                 170                 175

Tyr Arg Val Val Ser Val Leu Thr Val Leu His Gln Asp Trp Leu Asn
            180                 185                 190

Gly Lys Glu Tyr Lys Cys Lys Val Ser Asn Lys Gly Leu Pro Ser Ser
        195                 200                 205

Ile Glu Lys Thr Ile Ser Lys Ala Lys Gln Pro Arg Glu Pro Gln Val
    210                 215                 220

Tyr Thr Leu Pro Pro Ser Gln Glu Glu Met Thr Lys Asn Gln Val Ser
225                 230                 235                 240

Leu Thr Cys Leu Val Lys Gly Phe Tyr Pro Ser Asp Ile Ala Val Glu
                245                 250                 255

Trp Glu Ser Asn Gly Gln Pro Glu Asn Asn Tyr Lys Thr Thr Pro Pro
            260                 265                 270

Val Leu Asp Ser Asp Gly Ser Phe Phe Leu Tyr Ser Arg Leu Thr Val
        275                 280                 285

Asp Lys Ser Arg Trp Gln Glu Gly Asn Val Phe Ser Cys Ser Val Met
    290                 295                 300

His Glu Ala Leu His Asn His Tyr Thr Gln Lys Ser Leu Ser Leu Ser
305                 310                 315                 320

Leu Gly Lys

<210>  18
<211>  11
<212>  PRT
<213>  human

<220>
<221>  misc_feature
<223>  Fc-portion N-glycosilation motif

<400>  18

Arg Glu Glu Gln Phe Asn Ser Thr Tyr Arg Val
1               5                   10